# EUROPEAN PATENT SPECIFICATION

(11) **EP 0 650 527 B1**
(45) Date of publication and mention of the grant of the patent: **12.03.2003**
(21) Application number: 92922907.8
(22) Date of filing: 22.10.1992
(51) Int. Cl.: C12Q 1/68, C12N 15/31, A61K 39/02

(54) **IMPROVEMENT IN BORRELIA BURGDORFERI DIAGNOSIS AND PROPHYLAXIS**
VERBESSERUNGEN DER DIAGNOSE UND DER PROPHYLAXE VON BORRELIA BURGDORFERI
AMELIORATION DANS LE DIAGNOSTIC ET LA PROPHYLAXIE DE LA BORRELIOSE PROVOQUEE PAR BORRELIA BURGDORFERI

(30) Priority: 22.10.1991 US 779185
(43) Date of publication of application: 03.05.1995
(73) Proprietor: SYMBICOM AB, 431 83 Mölndal (SE)
(72) Inventor: BARBOUR, Alan, George, Newport Beach CA 92660 (US); BERGSTROM, Sven, S-Umea (SE); HANSSON, Lennart, S-435 43 Pixbo (SE)
(74) Representative: Plougmann & Vingtoft A/S
(86) International application number: US9208972
(87) International publication number: WO93008306

(56) References cited:
- EP-A- 0 418 827
- WO-A-90/04411
- WO-A-92/00055
- RESEARCH IN MICROBIOLOGY vol. 142, no. 5, 1991, PARIS, FRANCE pages 565 - 572 M. DEBUE ET AL. 'Detection of Borrelia burgdorferi in biological samples using the polymerase chain reaction assay'
- PROTEIN EXPRESSION AND PURIFICATION vol. 1, 1990, NEW YORK, USA pages 159 - 168 J.J. DUNN EET AL. 'Outer surface protein A (OspA) from the lyme disease spirochete, Borrelia burgdorferi: High level expression and purification of a soluble recombinant form of OspA'
- MOLECULAR MICROBIOLOGY vol. 3, no. 4, April 1989, pages 479 - 486 S. BERGSTR\M ET AL. 'Molecular analysis of linaer plasmid-encoded major surface proteins, OspA and OspB, of the lyme disease spirochaete Borrelia burgdorferi'
- JOURNAL OF CLINICAL MICROBIOLOGY vol. 29, no. 3, March 1991, WASHINGTON, DC, USA pages 524 - 532 P.A. ROSA ET AL. 'Polymerase chain reaction analyses identify two distinct classes of Borrelia burgdorferi' cited in the application
- JOURNAL OF CLINICAL MICROBIOLOGY vol. 28, no. 3, March 1990, WASHINGTON, DC, USA pages 566 - 572 D.H. PERSING ET AL. 'Detection of Borrelia burgdorferi infection in Ixodes dammini Ticks with the polymerase chain reaction'

## Description

The present invention relates to valuable and useful developments in diagnostics of and vaccines against Borrelia burgdorferi based upon new findings about new classes of Borrelia burgdorferi (from now on B. burgdorferi) and especially about DNA and peptide sequences from the B. burgdorferi strains ACA1 and Ip90 (the same as Iper90) and their relationship to B. burgdorferi strain B31, including special sequences from the three different strains of B. burgdorferi useful as primers in the PCR-DNA detection of B. burgdorferi in specimens from animals, including humans, a diagnostic kit for diagnosing Lyme disease in animals, including humans, DNA sequences from B. burgdorferi strains ACA1 and Ip90 encoding polypeptides related to the outer membrane protein OspA and OspB, a polypeptide encoded from ACA1 and Ip90, epitopes from OspA and OspB, and a vaccine against Lyme disease comprising one or more epitopes from OspA and OspB.

### BACKGROUND OF THE INVENTION

Lyme disease is a zoonosis caused by the tick-borne spirochaete B. burgdorferi. When a susceptible host is bitten by an ixodid tick, B. burgdorferi organisms enter the skin. In humans the initial skin manifestation is termed erythema chronicum migrans (ECM) whereas a long-standing infection of the skin produces acrodermatitis chronica atrophicans. The Borrelia organisms also enter the circulatory system of the host and are distributed to various organs, including the brain and joints. A secondary spread of the pathogens produces a variety of clinical syndromes, including lymphocytic meningoradiculitis, myocarditis and chronic arthritis. In many patients the infection of some tissues, particularly the brain and joints, persists for years and can be severely disabling. These forms of chronic Lyme disease are a consequence of the host's inability to rid itself of the infectious agent and perhaps the development of an autoimmune reaction.

Diagnosis of Lyme disease has chiefly been based on clinical evidence. The best marker during the primary stage of infection has conventionally been the presence of erythema chronicum migrans (ECM) but these skin lesions may not always develop or they may manifest atypically. Moreover, Lyme disease can be confused with other illnesses characterized by neurologic or arthritic manifestations. When clinical histories are incomplete, serologic testing with determination of antibody titers is the best conventionally laboratory method of diagnosis. Indirect fluorescent antibody (IFC) staining tests and enzyme-linked immunosorbent assays (ELISA) are used to detect total immunoglobulins or class-specific IgM and IgG antibodies to B. burgdorferi. ELISA is usually preferred because the procedures are more easily standardized and automated and because absorbance values can be statistically analyzed to give more objective results.

B. burgdorferi spirochaetes are helically shaped, motile cells with an outer cell membrane that surrounds a protoplasmic cylinder complex, consisting of the cytoplasm, the cell wall, the inner cell membrane and the flagella which are located not at the cell surface but in the periplasmic space between the outer cell membrane and the protoplasmic cylinder. The outer cell membrane and the flagella are assumed to play an important role in the host-parasite interactions during the disease and has been subjected to several investigations, identifying major surface-exposed proteins as important immunogens.

It has been shown that the earliest IgM antibodies formed against antigens of the B. burgdorferi strain B31, which was deposited in the American Type Culture Collection in 1983 with the accession number ATCC 35210, are directed against a genus-specific flagellar polypeptide termed flagellin having a molecular weight of 41 kd (18) and which reacts with monoclonal antibody H9724. IgG antibodies are also first directed to the 41 kd flagellin, but with advancing disease IgG antibodies form against other immunogens, especially against two abundant proteins with molecular weights of 31 kd and 34 kd. These two proteins, which have been denoted OspA (31 kd) and OspB (34 kd), have been found to be located at the B. burgdorferi surface and embedded in its outer fluid cell membrane. The OspA protein has been found to be less variable in its molecular weight and in its reactivity with monoclonal antibody H5332 (10), whereas the molecular weight of OspB proteins from different B. burgdorferi strains vary and the OspB proteins of different strains also show varying reactivity with two monoclonal antibodies against OspB (H6831 and H5TS) (9). The main variation among OspA proteins is found between isolates from Europe and the United States.

Conventional diagnostic tests for Lyme disease have used whole spirochaetal sonic extracts as test antigens in ELISA to detect antibodies to B. burgdorferi, but this test yields unsatisfactory low diagnostic sensitivity (20 to 60%) during the early stage of infection, possibly due to a slow and late-appearing antibody response and to the inclusion of irrelevant cross-reacting antigens in the whole-cell preparations. In addition, the use of whole cells as test antigens may result in the occurrence of false positive reactions. For example, among patients with syphilis and in areas where a closely related relapsing fever Borrelia spp. co-exist with B. burgdorferi, serologic differentiation of Lyme disease from tick-borne relapsing fever is difficult. Detection of IgG antibody to B. burgdorferi in later stages of infection can help in distinguishing Lyme disease from aseptic meningitis, multiple sclerosis, serum negative rheumatoid arthritis, juvenile rheumatoid arthritis, and Reiter's syndrome.

The antigen-antibody diagnostic approach has been found not to work as well in connection with Lyme disease as in connection with many other infectious diseases.

one of the reasons for this is that only a low number of spirochaetes is present and that the antigens in the outer membrane of the spirochaetes are hard to detect for the immune system of the infected organism.

Another reason is that the antibody response to the B. burgdorferi infection first arises weeks after the bite of the tick, and in many cases first after the patient has shown clinical signs of the disease.

It would be desirable to provide a diagnostic tool which is able to diagnose a B. burgdorferi infection at all stages, also, at very early stages even before the clinical signs of infections appear and the diagnostic tool being independent of the causative infective B. burgdorferi strain.

A most promising and sensitive method for diagnosing the Lyme disease agent is that of detecting a single B. burgdorferi organism by PCR amplification.

Nielsen S.L. et al. Molecular and Cellular Probes (1990) 4, 73-79, Detection of Borrelia burgdorferi DNA by the polymerase chain reaction, and Malloy, D.C. et al, Journal of Clinical Microbiology, June 1990, p. 1089-1093, Detection of Borrelia burgdorferi Using the Polymerase Chain Reaction, both disclose the use of DNA-sequences from only B. burgdorferi strain B31 in the preparation of primers useful in the PCR-DNA diagnostic of Lyme disease.

WO 91/06676 discloses the use of DNA primers associated with the SC plasmid in the diagnostic of Lyme disease, but does not disclose the sequence of the primers.

EP 421 725 A1 discloses nucleic acid probes useful for identifying B. burgdorferi in samples. The probes are designed from B. burgdorferi strains in The United States and Europe.

EP 445 135 discloses the use of a DNA fragment from the OspA gene from the B31 strain in PCR-DNA diagnostic of B. burgdorferi infection in mammals, including humans, as well as the use of immmunogenic polypeptides found to be antigenic when assessed with monoclonal antibodies directed against OspA in the preparation of a vaccine immunizing mammals, including humans, against Lyme disease. EP 445 135 discloses, inter alia, three contemplated epitopes which are small fragments of B31 OspA: Lys-Glu-Lys-Asn-Lys-Asp, Ser-Lys-Lys-Thr-Lys-Asp, and Lys-Ala-Asp-Lys-Ser-Lys. To the extent this is relevant, these fragments and their utility and use as epitopes is disclaimed in the present invention.

Rosa, P.A. et al., Journal of Clinical Microbiology, Mar. 1991, p.524-532, Polymerase Chain Reaction Analyses Identify Two Distinct Classes of Borrelia burgdorferi discloses PCR primers used in the identification.

Debue et al. *Research in Microbiology 142*, pp. 565-572 (1991) disclose two sets of PCR primers, one derived from OspA and the other from OspB of B. burgdorferi strain B31. The OspB primers were able to amplify the expected DNA in five of the six strains tested, but the OspA primers were only able to amplify the expected DNA in three of the six strains tested.

One object of the present invention is to provide non-immunological assays by providing nucleotide sequences which can hybridize with different strains of B. burgdorferi from different geographical regions so that a diagnostic tool for detecting B. burgdorferi spirochaetes independent of causative infective strain of B. burgdorferi is obtained. Another object is to provide novel DNA fragments related to B. burgdorferi. A further object of the invention is to provide antigenic polypeptides, an antigenic composition and a vaccine for immunizing animals, including humans, against Lyme disease substantially independent of the infective B. burgdorferi strain causing the disease.

### DESCRIPTION OF THE INVENTION

In one aspect, the invention relates to a method for detecting the presence of a *B. burgdorferi* organism in animals, including humans, the method comprising subjecting a specimen from the animal, such as a body fluid, such as blood, serum, cerebrospinal fluid, synovial fluid, pericardial fluid or urine, or a tissue biopsy, or, when the animal is an arthropod, the whole animal, to PCR-DNA analysis which either
A) comprises using a nucleotide primer sequence which comprises at least 11 nucleotides, the primer sequence being identical or substantially identical to a subsequence of at least two of three OspA and OspB DNA sequences derived from *B. burgdorferi* strains B31, Ip90 and ACA1, respectively, and, upon completion of the PCR DNA-analysis, identifying the detected *B. burgdorferi* organism as belonging to one of the classes I, II and III of *B. burgdorferi* of which strains B31, Ip90 and ACA1, respectively, are reference strains, or
B) comprises using a combination of at least two nucleotide primer sequences, each of which comprises at least 11 nucleotides, one sequence being identical or substantially identical to a sub-sequence of the OspA and OspB DNA from a first *B. burgdorferi* strain B31, another sequence being identical or substantially identical to a sub-sequence of the OspA and OspB DNA from another *B. burgdorferi* strain Ip90, and optionally a third sequence which is identical or substantially identical to a sub-sequence from the third *B. burgdorferi* strain ACA1 and, upon completion of the PCR DNA-analysis, identifying the detected *B. burgdorferi* organism as belonging to one of the classes I, II and III of *B. burgdorferi* of which strains B31, Ip90 and ACA1, respectively, are reference strains,
the substantial identity being such that the said primer sequence will hybridize to the sub-sequence under conventional hybridization conditions (reference Sam Brooks), or under high stringency hybridization conditions comprising hybridization at 67°C in 2xSSC and final washing at 67°C in 1xSSC, or under medium high stringency hybridization conditions comprising hybridization at 67°C in 3xSSC and final washing at 67°C in 1xSSC.

It has previously been shown that B. burgdorferi strains of different geographical origin differ in DNA sequence profiles, and until now two classes of B. burgdorferi have been disclosed (36).

According to the present invention the OspA and OspB genes from two B. burgdorferi strains ACA1 and Ip90 have been sequenced and compared to the OspA and OspB gene sequence from strain B31, previously sequenced (14). The differences seen when comparing the sequences from B. burgdorferi strains B31, ACA1 and Ip90 indicate that B. burgdorferi may be placed into three different classes or species I, II and III, using B31, Ip90 and ACA1 as reference strains, instead of placing B. burgdorferi into two classes.

The method proves a diagnostic tool which may be used to diagnose B. burgdorferi infection at all stages, even before the patient shows any clinical signs of Lyme disease, because the B. burgdorferi spirochaetes are detected instead of the antibodies towards the spirochaetes. Also, the method is able to detect even a single B. burgdorferi spirochaete and therefore the sensitivity of the method is very high.

Furthermore, the method is useful in diagnosing whether a Ixodes tick is a vector for B. burgdorferi, which is of especially interest in areas with endemic B. burgdorferi infections.

The B. burgdorferi strain ACA1 is a Swedish isolate from the tick vector I. ricinus, and the B. burgdorferi strain Ip90 is an isolate from the Soviet Union from a region in which the tick vector is I. persulcatus. The known B. burgdorferi strain B31 is a North American isolate from the tick vector I. damminii.

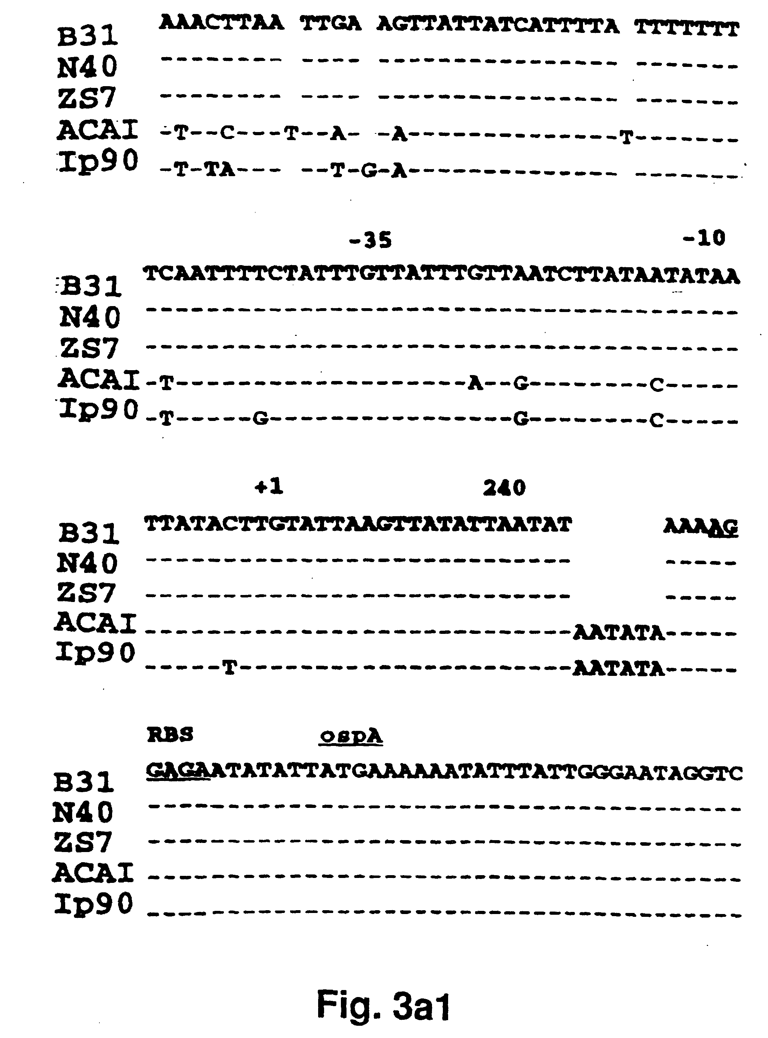

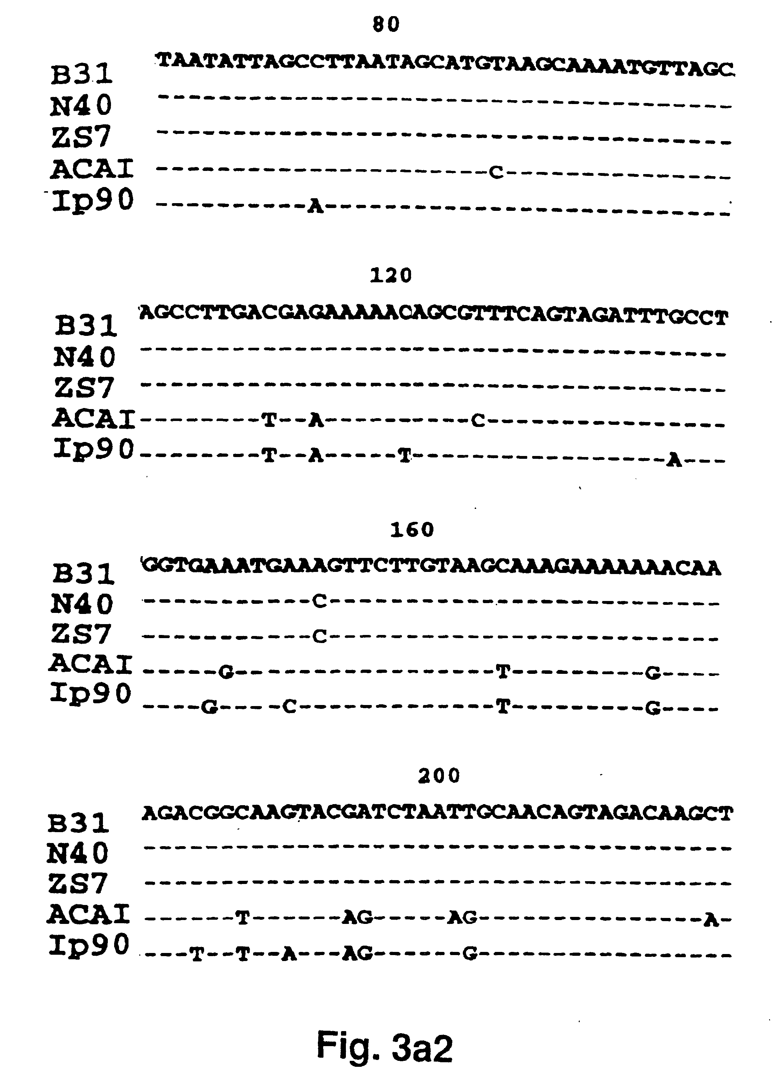

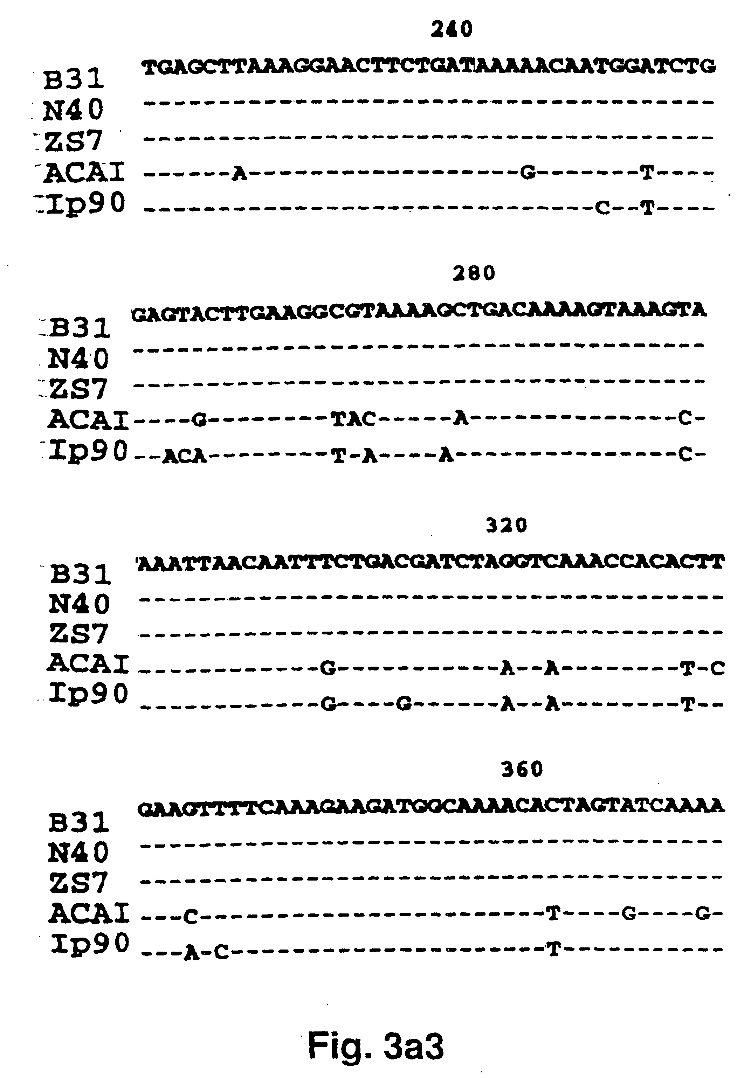

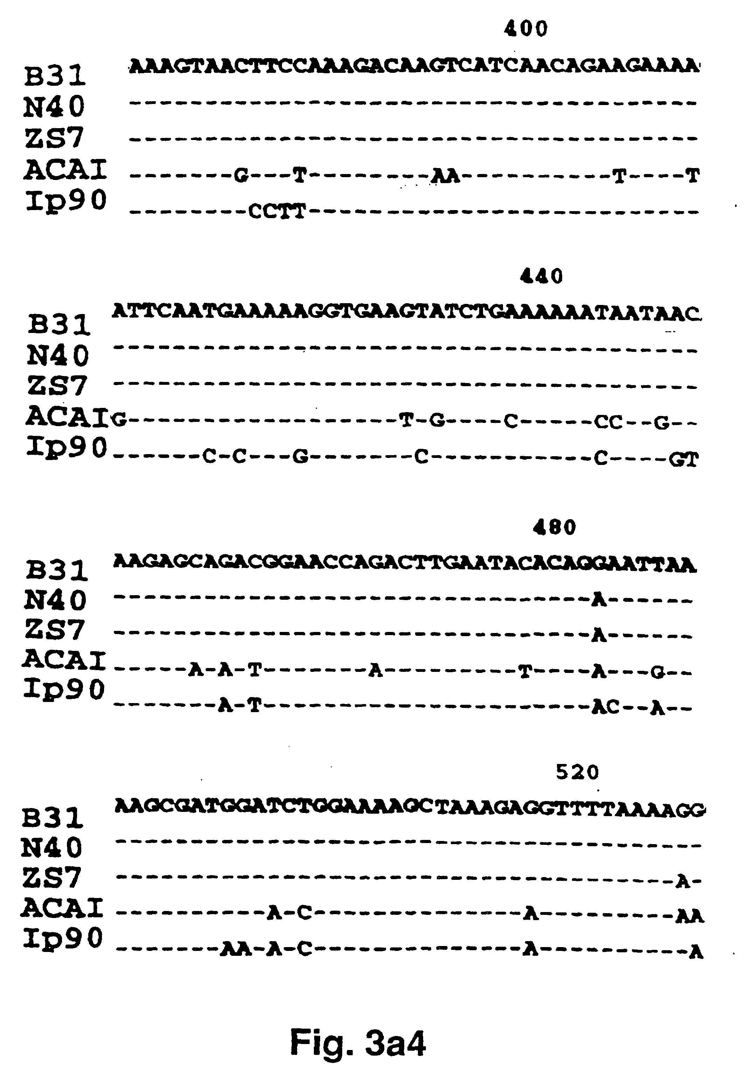

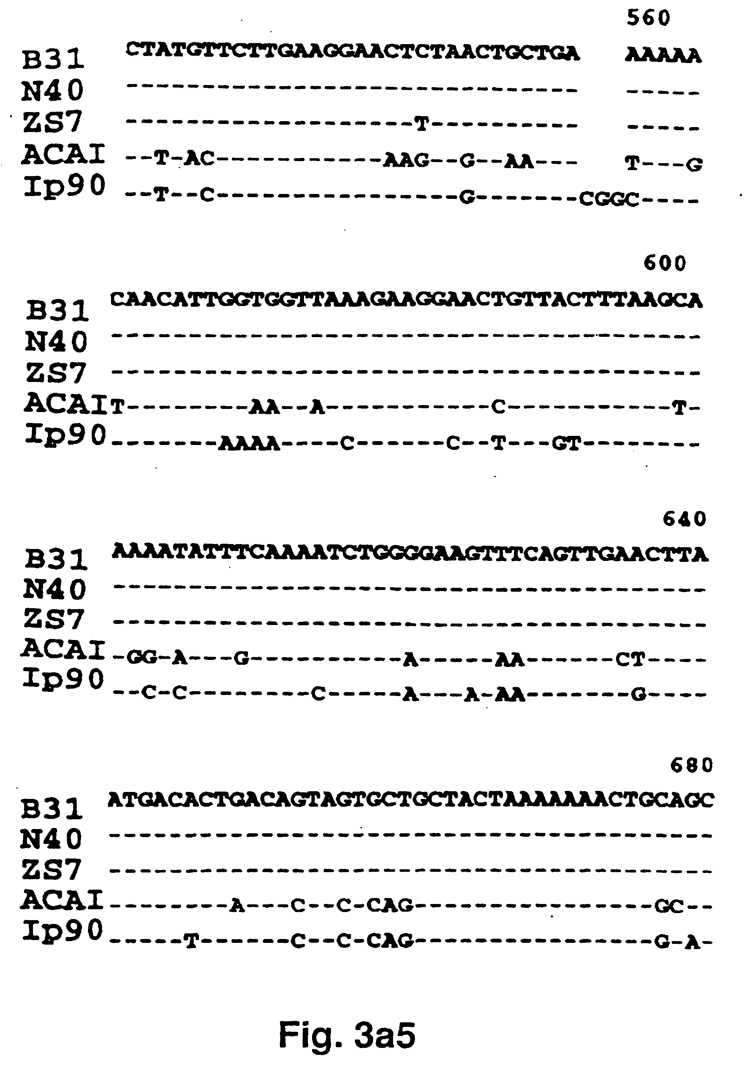

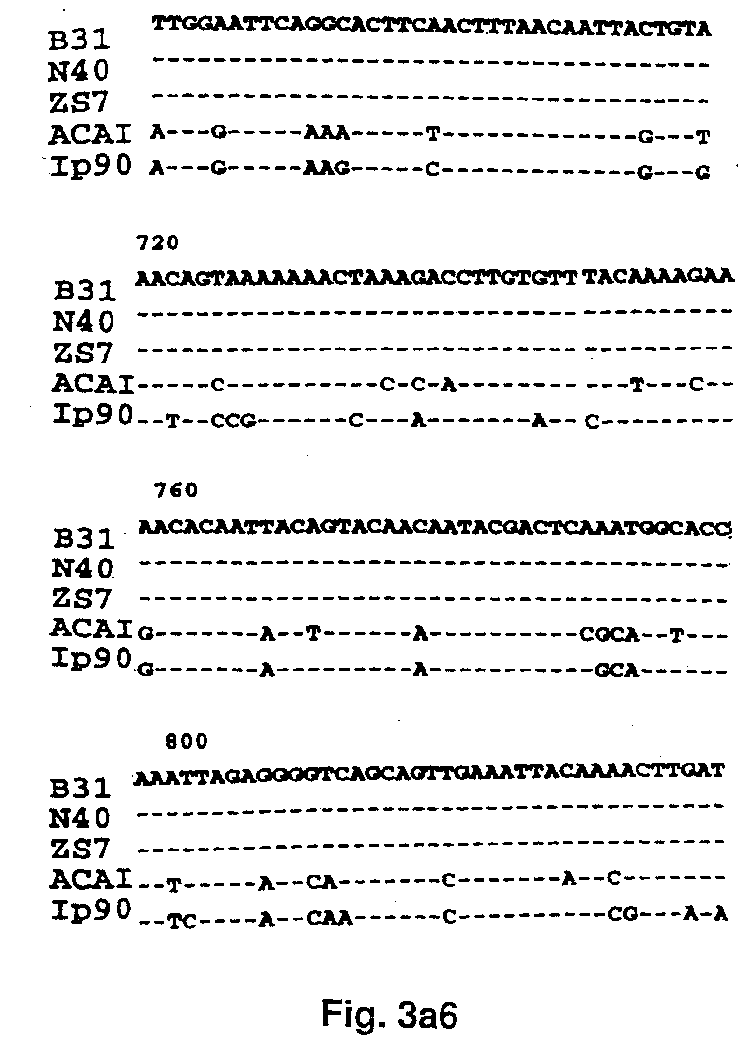

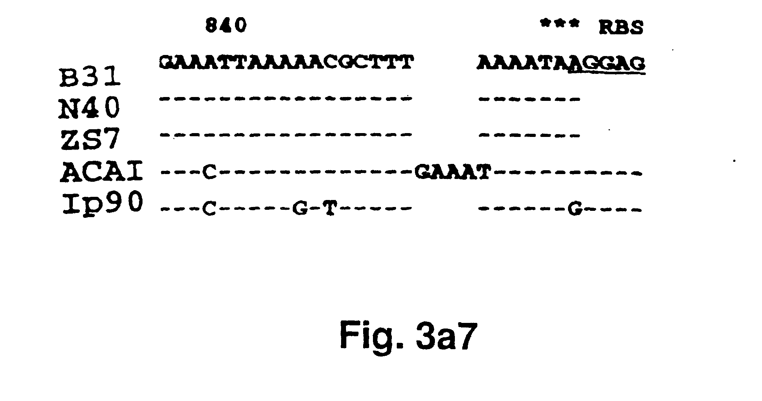

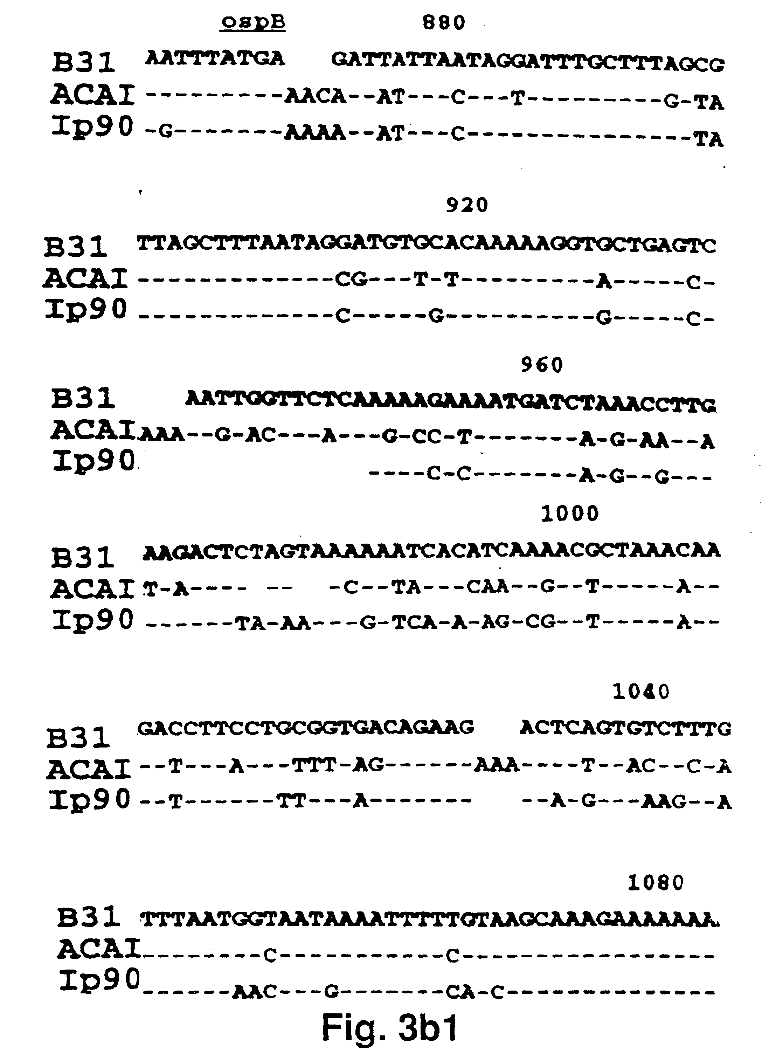

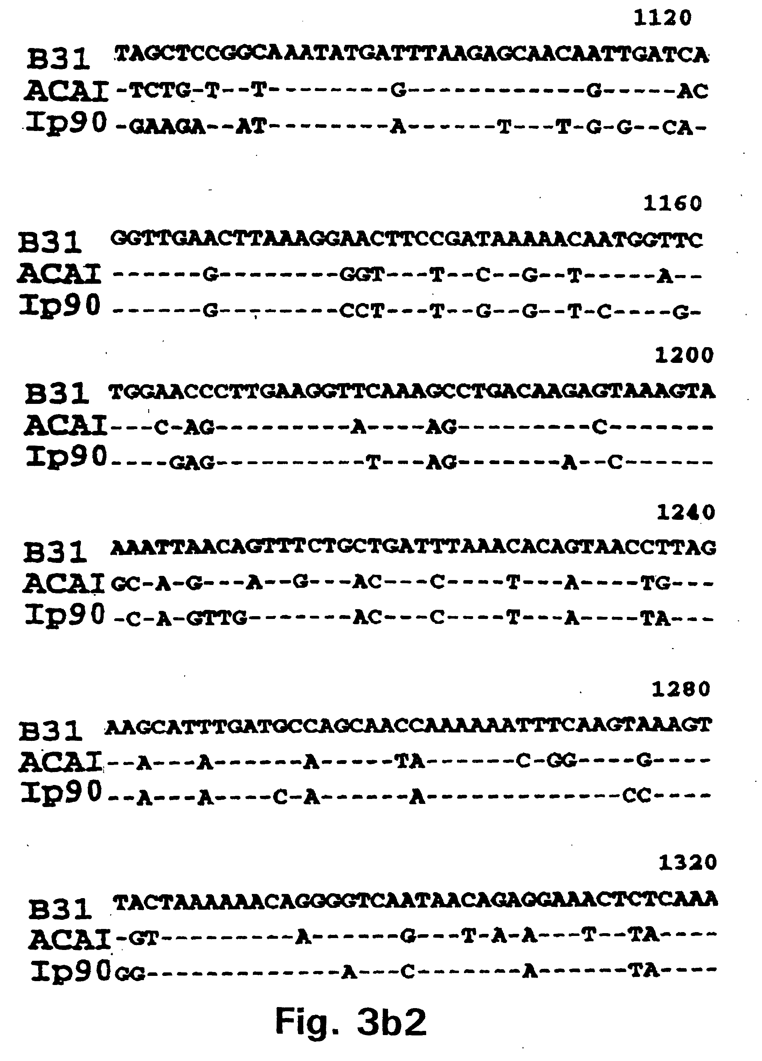

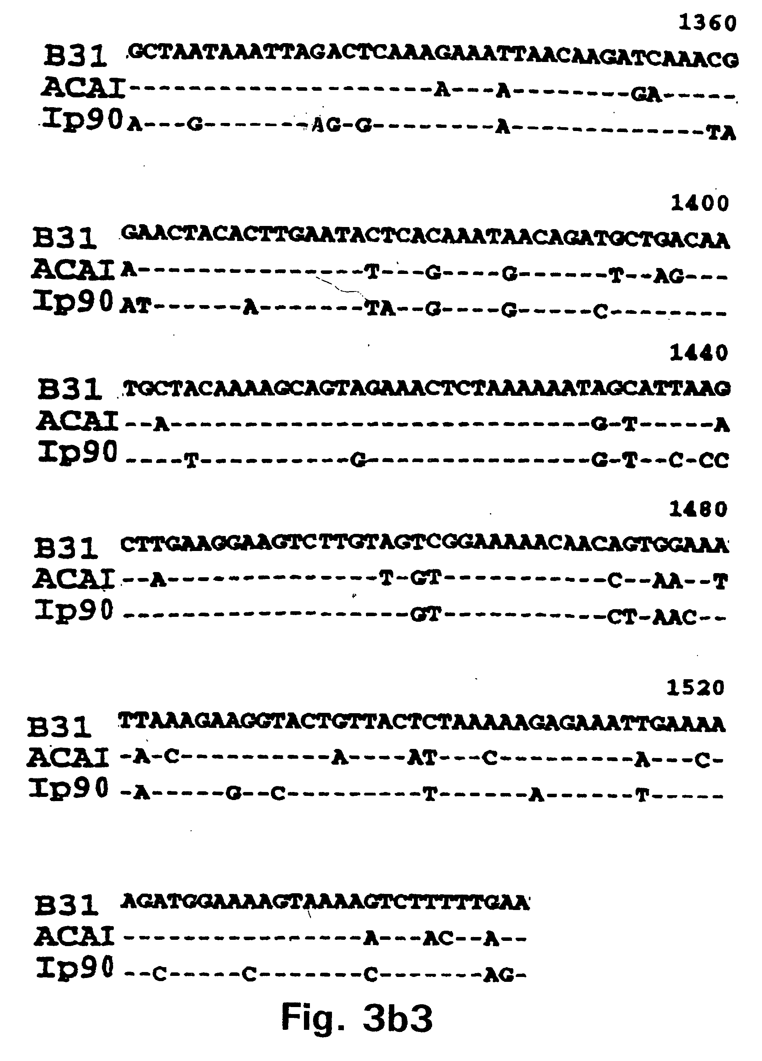

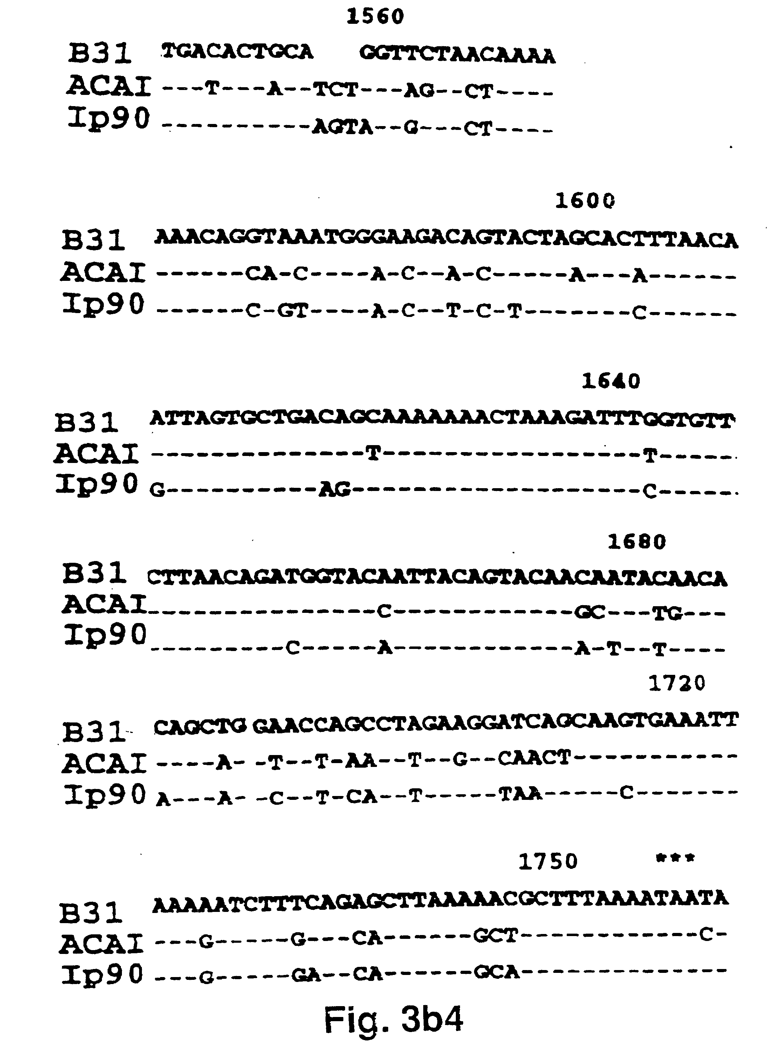

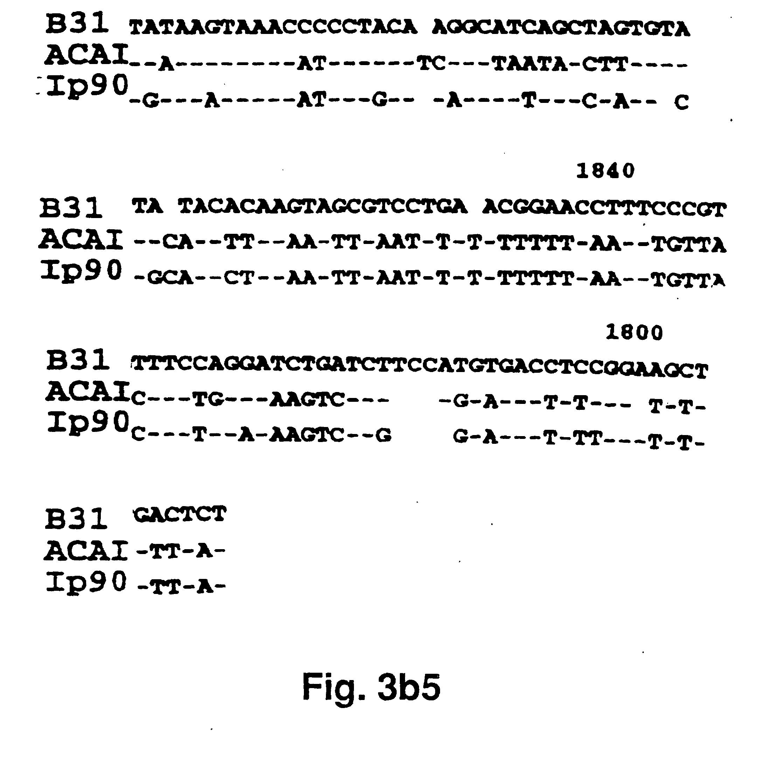

Comparison between the known B31 sequence and the two novel sequences from ACA1 and Ip90, respectively, reveals that there are several regions having conserved sequences either totally or with a few mismatches of nucleotides in the regions, Fig. 3.

As mentioned above, the strains discussed herein are from different geographical regions. According to a further aspect of the invention, primers are provided which can detect *B. burgdorferi* of all classes by the PCR amplification method, by using nucleotide sequences from regions having the same or substantially the same DNA-sequence in all the three strains, therefore the invention relates to a method wherein the PCR-analysis either
A) uses a nucleotide primer sequence which comprises at least 11 nucleotides, the primer sequence being identical or substantially identical to a sub-sequence of at least two of the three OspA and OspB DNA sequences derived from *B. burgdorferi* strains B31, Ip90 and ACA1, respectively, with the proviso that the primer sequence is different from 5'-CTCCGGCAAATATGATTTAAGAGCA-3' and 5'-GGTTGCTGGCATCAAATGCTTCTA-3', or
B) uses a combination of at least two nucleotide primer sequences, each of which comprises at least 11 nucleotides, one sequence being identical or substantially identical to a sub-sequence of the OspA and OspB DNA from a first *B. burgdorferi* strain B31, another sequence being identical or substantially identical to a sub-sequence from the OspA and OspB DNA from another *B. burgdorferi* strain Ip90, and optionally a third sequence which is identical or substantially identical to a sub-sequence from the third *B. burgdorferi* strain ACA1,
the substantial identity being as defined above.

In the present context the term "substantially identity" is intended to indicate that the sequences only have one or a few mismatches, and that these mismatches will not interfere with the annealing of the primer sequence in question to its target sequence with sufficient specificity. A more precise definition is based on hybridization: A primer sequence which is substantially identical with a particular sub-sequence in the context of the present invention will hybridize to the sub-sequence under defined hybridization conditions. Several sets of conventional hybridization conditions relevant in the present context are described in (52). For a primer, however, a rather high degree of specificity is often required, and this is reflected in the above specification of high stringency hybridization conditions comprising hybridization at 67°C in 2xSSC and final washing at 67°C in 1xSSC, or medium high stringency hybridization conditions comprising hybridization at 67°C in 3xSSC and final washing at 67°C in 1xSSC.

The primers may, in principle, be shorter than the 11 nucleotides mentioned above, but this is normally not preferred for specificity reasons. On the contrary, it is normally preferred that the primers are somewhat longer, such as comprising at least 12 or 13 nucleotides, preferably at least 15 nucleotides, and in many cases about 18 nucleotides or even higher.

The most interesting primers for use in a general PCR-DNA detection of B. burgdorferi are, of course, primers which are especially capable of amplifying DNA from a B. burgdorferi irrespective of the particular strain or species of the B. burgdorferi. Primer sequences of particular interest in this connection are primers corresponding to subsequences which are identical or substantially identical in all three of the species I, II and III, such as in all three of the strains B31, Ip90 and ACAl. Primers fulfilling this condition are, e.g. primers which are fragments comprising a sequence which is identical or substantially identical to one of the following sequences (or one of their complementary sequences):

5′-GTATTAAGTTATATTAATAT-3′    (SEQ.ID.NO. 1, bp 123-142)

5′-AAAAGGAGAATATATTATGA-3′    (SEQ.ID.NO. 1, bp 584-607)

5'-AAAAATATTTATTGGGAATA-3'    (SEQ.ID.NO. 1, bp 776-794)

5′-GGAAAAGCTAAAGAGGTTTTAAAA-3′ (SEQ.ID.NO. 1, bp 806-817)

5′-ACTTCAACTTTAACAATTA-3′ (SEQ.ID.NO. 4, bp 85-104)

5′-AATAAGGAGAATTTATGA-3′     (SEQ.ID.NO. 4, bp 111-130)

5'-AAAAAAACTAAA-3'                    (SEQ.ID.NO. 4, bp 948-965).

As will be seen from Fig. 3 which shows the DNA sequences (a single strand of the double-stranded DNA shown) of the B. burgdorferi strains B31, ACA1, and Ip90, the above specific DNA fragments represent regions showing full or almost full identity between the three strains.

While PCR analysis can be performed using a single primer, it is, of course, normally more advantageous to utilize the exponential amplification obtained when using a set of primers amplifying complementary strands such as is normal in PCR. Therefore, in a preferred embodiment of this aspect of the invention, a set of primers is used, the first primer of the set being a primer as defined above, the second primer comprising a sequence which is identical or substantially identical to a subsequence of a strand complementary to OspA or OspB DNA from at least one of B. burgdorferi strains B31, Ip90 and ACA1 as shown in Fig. 3 herein, the subsequence of the complementary strand being downstream of the first primer in relation to the direction of expression of the first primer, the substantial identity being as defined above. When a set of primers is used, it is normally preferred that they have substantially the same degree of identity with their target sequences. The distance between the primers in the set may be from 25 to 300 bp, such as 50 to 200 bp.

While the embodiments discussed above focus on the establishment of a PCR analysis which will detect DNA from a B. burgdorferi spirochaete irrespective of which strain it is, another interesting aspect of the invention is a PCR analysis making it possible to detect the presence of a B. burgdorferi spirochaete in a specimen with strain specificity, in other words to detect specifically a B. burgdorferi of one of the now-identified three main classes or species.

It is of interest to be able to know exactly the class and possibly also the strain responsible for an infection because it seems as if different classes of B. burgdorferi relate to different symptom complexes. In Scandinavia where B. burgdorferi from class III, represented by ACAI is common the symptoms arising from B. burgdorferi infections are more often neurological symptoms compared to what is seen in other areas. Contrary to this arthritis resulting from B. burgdorferi infection appears more often in USA where B. burgdorferi class I, represented by B31, is common than elsewhere.

In this aspect, the specimen is subjected to PCR-DNA analysis using as a primer a DNA sequence which comprises at least 11 nucleotides, the sequence being identical or substantially identical to a subsequence of OspA and OspB DNA from one of the B. burgdorferi species I, II and III which subsequence is different from any subsequence of the two other species, the difference being such that the sequence will not anneal to DNA from the two other species, the substantial identity being as defined above.

Also, with respect to species or strain specifically detection of B. burgdorferi it is advantageous to use a set of primers, the first primer of the set being a primer as defined above, the second primer comprising a sequence which is identical or substantially identical to a subsequence of a strand complementary to ospA or OspB DNA from the same B. burgdorferi species, preferably of the same strain as the first primer, the subsequence of the complementary strand being downstream of the first primer in relation to the direction of expression of the first primer, the substantial identity being as defined above.

In a preferred embodiment of the invention the subsequence of the first primer is identical or substantial identical to a subsequence of OspA and OspB from one of the B. burgdorferi strains B31, ACAI and Ip90 as shown in Fig. 3 and the second primer being as defined above.

Again, the length of the primer is preferably at least 12, more preferably at least 13 or 15 and often about 18, such as explained above.

The difference in sequences which is decisive in this type of PCR according to the invention can be identified directly from Fig. 3 and confirmed by simple experiments.

Especially, the first primer is a DNA sequence of 15-25 nucleotides which differs from any subsequence of the two other strains in at least 4 nucleotides per 20 nucleotides of the primer, preferably the differences being in 5 nucleotides per 20 nucleotides.

When differences are present in 4 or more nucleotides per 20 nucleotides of a primer a minimum of cross-reaction between the primer and a B. burgdorferi strain from another species is possible irrespective of the stringency hybridization conditions.

In order to detect an exact B. burgdorferi species or strain without knowing the species forehand, it is preferred to use a set of primers wherein each primer comes from a different species or strain and differs from any subsequences of the other species and strains in at least 4 nucleotides per 20 nucleotides of primers. In a preferred embodiment 3 sets of primers are used, the sets comprising
- a first primer of the first set which is a DNA sequence of 15-25 nucleotides identical or substantially identical to a subsequence of the DNA from B. burgdorferi strain B31 shown in figure 3 which differs from any subsequence of the DNA sequence from the two other B. burgdorferi strains shown in figure 3 for at least 4 nucleotides per 20 nucleotides of the primer, preferably for at least 5 nucleotides per 20 nucleotides,
- a first primer of the second set which is a DNA sequence of 15-25 nucleotides identical or substantially identical to a subsequence of the DNA from B. burgdorferi strain ACA1 shown in figure 3 which differs from any subsequence of the DNA sequence from the two other B. burgdorferi strains shown in figure 3 for at least 4 nucleotides per 20 nucleotides of the primer, preferably for 5 nucleotides per 20 nucleotides
- a first primer of the third set which is a DNA sequence of 15-25 nucleotides identical or substantially identical to a subsequence of the DNA from B. burgdorferi strain Ip90 shown in figure 3 which differs from any subsequence of the DNA sequence from the two other B. burgdorferi strains shown in figure 3 for at least 4 nucleotides per 20 nucleotides of the primer, preferably for 5 nucleotides per 20 nucleotides,
and for each set, a second primer which is a subsequence of the DNA sequence of the complementary strand of the same strain as the first primer of the set, the subsequence of the complementary strand being downstream of the first primer in relation to the direction of expression of the first primer, the second primer preferably differing from any subsequences of the DNA sequence of the two other strains.

Again, it is preferred to use a set of 2 primers for each primer defined above, the second primers coming from the complementary strand of the same B. burgdorferi species or strain.

Species specifically diagnostic is especially interesting if all the strains from a particular B. burgdorferi species is detected and at the same time that no other strains are detected, i.e. that both the sensitivity and the specificity are high. This will most probable occur if the primers are subsequences that are well conserved within a species but differs from any subsequences from any other strains. OspA sequences are generally better conserved within species than OspB, therefore it is preferred to use primers that are subsequences identical or substantially identical to subsequences from the DNA sequences encoding OspA, such as those shown in figure 3 coding for OspA.

Preferably, the first primers of sets for species or strain specifically detection of B. burgdorferi are selected from 
AACAATGGATCTGGAGTA (SEQ. ID. NO. 1, bp 326-343)
AACAACGGTTCTGGAACA (SEQ. ID. NO. 7, bp 332-349)
GTCAAGAAAAGTAAGTTCTA (SEQ. ID. NO. 4, bp 456-475)
 and

CTCTAACTGCTGAAAAAAC (SEQ. ID. NO. 1, bp 627-645)
AAGTAGCTAATGATAAAGT (SEQ. ID. NO. 4, bp 635-653)
CTCTAGCTGCTGACGGCAAAAC (SEQ. ID. NO. 7, bp 633-654)

The PCR analysis using the special primers discussed herein is performed in accordance with usual PCR technique such as described in the literature, e.g. (51). Thus, for the detection, the primer may be labeled, such as with radioactive labels, fluorescent dyes, and biotin, or labeled nucleotide triphosphates (e.g. labeled with thymidine) can be included in the PCR reaction to label the PCR amplification product.

The PCR primers used according to the invention may be prepared using well-known methods. Thus, they may be prepared by oligonucleotide synthesis, or they may be prepared by fragmentation of a larger nucleotide sequence using suitable restriction enzymes. The labelling of the primers can be performed by methods well-known per se.

As is conventional, the PCR reagents can be included in suitable PCR kits.

While the above discussion emphasizes the use of a single kind of primer capable of detecting all the strains of B. burgdorferi in a universal test, such a test can, of course, also be established by using a combination of different primers, comprising, for each of the strains of B. burgdorferi to be detected, a type of primer unique to the strain. Preferably, for specific detection the PCR kit should include a set of 2 primers for each species to be detected, e.g. a kit having 3 sets of 2 primers in appropriate amounts together with other PCR reagents.

Using the PCR analysis method or kit as described above, it is possible to diagnose Lyme disease as well as pre-clinical borreliosis in mammals, including humans, independent of the strain of B. burgdorferi that have caused the disease.

In another embodiment, the present invention relates to important novel DNA fragments and their use.

DNA fragments of the invention are fragments encoding the OspA and OspB of B. burgdorferi of the Swedish strain ACA1 and of the Soviet strain Ip90 or any modifications of said sequence encoding a polypeptide which is functionally equivalent to OspA and OspB from ACA1 and Ip90, respectively.

The term "functional equivalent" is intended to include all immunogenically active substances with the ability of evoking an immune response in animals, including humans to which the equivalent polypeptide has been administered, e.g. as a constituent of a vaccine which immune response is similar to the immune response evoked by the OspA and OspB. Thus, equivalent polypeptides are polypeptides capable of conferring immunity to Lyme disease.

OspA and OspB are outer surface proteins in B. burgdorferi which are encoded by DNA-sequences found within the same operon. The genes encoding OspA and OspB are located on a double stranded linear plasmids.

One embodiment of the invention is a DNA fragment comprising a nucleotide sequence as shown in Fig. 3 herein for ACA1, or for Ip90, a subsequence thereof which comprises at least 15 nucleotides and which hybridizes to said nucleotide sequence under high stringency hybridization conditions comprising hybridization at 67°C in 2xSSC and final washing at 67°C in 1xSSC, or under medium high stringency hybridization conditions comprising hybridization at 67°C in 3xSSC and final washing at 67°C in IxSSC, or under low stringency conditions such as 6 x SSC and 67°C and subsequent wash at 4 x SSC and 67°C, or a homologue of said nucleotide sequence which encodes a polypeptide identical to or substantially identical to a polypeptide encoded by said nucleotide sequence and which hybridizes to said nucleotide sequence under high or medium high or low stringency hybridization conditions as defined above.

An example of a hybridization procedure is described in connection with the examples.

In connection with the DNA fragment aspect of the present invention, the term "subsequence" is used to denote a nucleotide sequence which is derived from the nucleotide sequence shown by modifications e.g. of the types described below, and which has retained a characteristic nucleotide sequence thereof, the characteristic nucleotide sequence being a sequence which is capable of hybridizing to the nucleotide sequence in question under the conditions as explained herein. Typically, the subsequence is a part of the DNA fragment, that is, the subsequence is a number of nucleotides shorter than the DNA fragment.

In accordance with what is explained above, the DNA fragment of the invention may be one which has been modified by substitution, addition, insertion, deletion, transposons or rearrangement of one or more nucleotides in the sequence, e.g. for the purpose of establishing a sequence which, when expressed in a suitable host organism, results in the production of a polypeptide which has a substantial similarity to the OspA or OspB protein or a fragment thereof, and which has retained the immunological activity of the protein or the fragment.

The complete fragment shown in Fig. 3 comprises fragments coding for OspA protein and for OspB protein. One particular aspect of the invention is a DNA fragment as defined above comprising a nucleotide sequence starting at nucleotide 37 and terminating at nucleotide 859 of the nucleotide sequence shown in Fig. 3 herein encoding a cell membrane protein from B. burgdorferi, strain ACA1 (corresponding to SEQ. ID. NO. 4, bp 127-948), or from B. burgdorferi, strain Ip90, (corresponding to SEQ. ID. NO. 7, bp 125-949) said protein being designated OspA, or a subsequence or homologue of said nucleotide sequence, the nucleotide numbering being based on the B31 numbering in the manner shown in Fig. 3. Another particular aspect of the invention is a DNA fragment comprising a nucleotide sequence starting at nucleotide 865 and terminating at nucleotide 1855 of the nucleotide sequence shown in Fig. 3 herein encoding a cell membrane protein from B. burgdorferi, strain ACA1 (corresponding to SEQ. ID. NO. 4, bp 962-1861) , or from B. burgdorferi, strain Ip90 (corresponding to SEQ. ID. NO. 7, bp 959-1843) , said protein being designated OspB, or a subsequence or homologue of said nucleotide sequence, the nucleotide numbering being based on the B31 numbering in the manner shown in Fig. 3.

The modifications or homologues of the DNA fragments encoding OspA, OspB or a part thereof falling within the definitions herein above may be prepared, e.g. by subjecting the native DNA fragments to mutagenization, e.g. by treatment with ultraviolet radiation, ionizing radiation or a chemical mutagen such as mitomycin C, 5-bromouracil, methyl-methane sulphonate, nitrogen mustard or a nitrofuran, in particular so as to alter some of the properties of the gene product expressed from the mutagenized sequence substantially without changing the immunologic activity of the gene product. Especially, site-directed mutagenesis or directed mutagenesis is useful. All of these techniques are described in textbooks in the field, e.g. in (52) and (53).

The DNA sequences shown in Fig. 3 are discussed in detail in Example 1 herein.

Especially interesting DNA fragments are fragments of at least 15 nucleotides which encode immunologically active parts of OspA or Osp B, i.e. the antigenic determinants or epitopes of OspA, that is, in particular, polypeptides which are capable of interacting with immunocompetent cells to elicit an immune response against B. burgdorferi as well as lipidated OspA and OspB or lipidated antigenic determinants or epitopes of OspA or OspB. For vaccine purposes the use of lipidated polypeptides have proven a very interesting alternative to the use of non-lipidated polypeptides together with adjuvants.

In WO 90/04414 by the same applicant as the present application, DNA encoding OspA and OspB also encoding a cleavage site of a lipoprotein signal peptidase is disclosed. It has now been demonstrated that naturally occurring OspA and OspB are lipoproteins by ³H-palmitic acid labelling.

It is considered advantageous to immunize mammals with lipidated OspA or OspB or immunogenic determinant thereof because an antibody response is seen also in cases where no adjuvant is used in the vaccine. When non-lipidated polypeptides are used both for priming and boosting a very little and sometimes not even recognizable antibody response appears. If lipidated polypeptides are used for priming an immune response, non-lipidated polypeptides may be used for boosting and are indeed very effective boosting antigens. In the present context the term "priming" means the initial immunization, whereas the term "boosting" means the optional additional immunizations administered at certain intervals after the priming dose to sustain the immune response to the antigen. The boosting dose is often smaller than the priming dose.

It is well known that some antigens are able to trigger an autoimmune response in some individuals but not necessarily in all individuals when their immune system is presented to the antigen. When preparing a polypeptide for use as an antigen in a vaccine it is advantageous to delete sequences that are suspected or proved to be autoimmunic epitopes. Therefore, a further aspect of the present invention is a DNA fragment a polypeptide wherein epitopes responsible for evoking autoimmunity in animals, including humans, when administered to the animal have been deleted.

In the OspA and OspB polypeptides encoded by the DNA fragments according to the invention, polypeptide fragments comprising sequences selected from the following: 
LVSKEKNKDGKYDL (SEQ.ID.NO. 2, residues 41-54),
LVSKEKDKDGKYSL (SEQ.ID.NO. 5, residues 41-54),
 
KGTSDKNNGSGV (SEQ.ID.NO. 2, residues 64-75),
KGTSDKTNGSGV (SEQ.ID.NO. 5, residues 64-75),
KGTSDKNNGSGT (SEQ.ID.NO. 8, residues 64-75),
 
LEGVKADKSKVKL (SEQ.ID.NO. 2, residues 76-88),
LEGTKDDKSKAKL (SEQ.ID.NO. 5, residues 76-88),
LEGEKTDKSKAKL (SEQ.ID.NO. 8, residues 76-88),
 
KKVTSKDKSSTEEK (SEQ.ID.NO. 2, residues 11-125),
RKVSSKDKTSTDEM (SEQ.ID.NO. 5, residues 112-125),
KKVTLKDKSSTEEK (SEQ.ID.NO. 8, residues 112-125),
 
KKTKDLVFTKEN (SEQ.ID.NO. 2, residues 230-241),
KKTTQLVFTKQD (SEQ.ID.NO. 5, residues 230-241),
RKTKNLVFTKED (SEQ.ID.NO. 8, residues 231-242),
 
QYDSNGTKLEGS (SEQ.ID.NO. 2, residues 247-258),
KYDSAGTNLEGT (SEQ.ID.NO. 5, residues 247-258),
KYDSAGTNLEGK (SEQ.ID.NO. 8, residues 248-259),
 
AVEITKLDEIKNALK (SEQ.ID.NO. 2, residues 259-273),
AVEIKTLDELKNALK (SEQ.ID.NO. 5, residues 259-273),
AVEITTLKELKDALK (SEQ.ID.NO. 8, residues 260-274),
 
DLNLEDSSKKSHQNAK    (SEQ.ID.NO. 3, residues 31-46),
DQEIINSDNTPKDSKK    (SEQ.ID.NO. 6, residues 33-48),
DQDVEDLKKDQKDDSK    (SEQ.ID.NO. 9, residues 28-43),
 
KIFVSKEKNSSGK (SEQ.ID.NO. 3, residues 64-76),
KIFVSKEKNSAGK (SEQ.ID.NO. 6, residues 66-78),
EIFISKEKNEDDK (SEQ.ID.NO. 9, residues 61-73),
 
KPDKSKVKLTVSAD (SEQ.ID.NO. 3, residues 105-118),
KADKTKVAMTIADD (SEQ.ID.NO. 6, residues 107-120),
KADKSKVTMLVSDD (SEQ.ID.NO. 9, residues 102-115),
 
KKTGKWEDSTSTL (SEQ.ID.NO. 3, residues 234-246),
KKTATWNETTNTL (SEQ.ID.NO. 6, residues 237-249),
KKTAVWNDTSSTL (SEQ.ID.NO. 9, residues 232-244),
 
KNLSELKNALK (SEQ.ID.NO. 3, residues 286-296),
KDLAALKAALK (SEQ.ID.NO. 6, residues 289-299),
KDLEALKAALK (SEQ.ID.NO. 9, residues 284-294)
 have been identified by computer analysis to be potential epitopes. Thus, such fragments and polypeptide fragments comprising sequences which are at least 70%, preferably at least 80% and more preferably at least 90% homologous with any of the above sequences and capable of interacting with immunocompetent cells to elicit an immune response against B. burgdorferi, said polypeptide fragment or fragments being smaller in size than naturally occurring OspA or OspB, are considered especially important antigenic/immunogenic polypeptide fragments which will be valuable to elicit immune responses to B. burgdorferi. Accordingly, DNA fragments encoding these polypeptide fragments are especially interesting DNA fragments according to the invention.

The DNA fragments according to the invention, including the DNA fragments illustrated in Fig. 3 or a subsequences of said fragment may be derived by screening B. burgdorferi for nucleotide sequences hybridizing to a DNA probe prepared on the basis of the full or partial nucleotide sequence shown in Fig. 3. Further, the DNA fragment sequence may be a synthetic sequence, i.e. a sequence which is prepared according to standard procedures, e.g. as described in Matthes et al., 1984 (29).

The DNA fragment of the invention may be used for the production of OspA, OspB or parts thereof, especially immunologically active parts thereof. For this purpose, conventional recombinant DNA techniques may be employed. Thus, techniques comprising inserting the DNA fragment of the invention or one or more parts thereof into a suitable expression vector, transforming a host organism with the vector, cultivating the organism under conditions allowing expression of the inserted sequence and harvesting the resulting gene product, OspA or a part thereof, will be useful. Any of these procedures may be carried out by standard methods such as those disclosed in Maniatis et al., 1982 (30).

In order to prepare lipidated polypeptides it is necessary to use a DNA fragment which encodes a polypeptide comprising a lipoprotein signal peptide recognised by signal peptidase.

Consequently, the present invention further relates to a DNA fragment comprising a lipoprotein signal peptide recognized by signal peptidase, such as lipoprotein signal peptide II (SPaseII) especially, those DNA fragments wherein the lipoprotein signal peptide comprises a sequence in the C-terminal region recognized by signal peptidase II, eg. the sequences described by von Heijne, 1989 (63).

In a preferred embodiment a DNA fragment according to the invention is a DNA fragment encoding a lipoprotein signal peptide comprising the following sequence L-y-x-C in the C-terminal region, where y and x may be independent and each designate a small, neutral amino acid, such as isoleucine, alanine and glycine.

The lipoprotein signal peptide comprises preferably at least 10, such as at least 13 amino acids. In a preferred embodiment the DNA fragment the lipoprotein signal peptide comprises from 16 to 35 amino acids, such as from 16 to 29 amino acids.

Suitable expression vectors for the production of OspA, OspB or a part thereof are vectors which is capable of replicating in a host organism when transformed therein. The vector may either be one which is capable of autonomous replication, such as a plasmid, or one which is replicated with the host chromosome, such as a bacteriophage. Examples of suitable vectors which have been widely employed are pBR322 and related vectors as well as pUC vectors and the like. Examples of suitable bacteriophages include M13 and lambda.

The organism harbouring the vector carrying the DNA fragment shown in Fig. 3 or part thereof may be any organism which is capable of expressing said DNA fragment. The organism is preferably a microorganism such as a bacterium. Gram-positive as well as gram-negative bacteria may be employed. Especially a gram-negative bacterium such as E. coli is useful, but also gram-positive bacteria such as B. subtilis and other types of microorganisms such as yeasts or fungi or other organisms conventionally used to produce recombinant DNA products may be used.

In order to produce lipidated polypeptides the host harbouring the vector is to be a host which is capable of expressing lipidated polypeptides, such as E. coli.

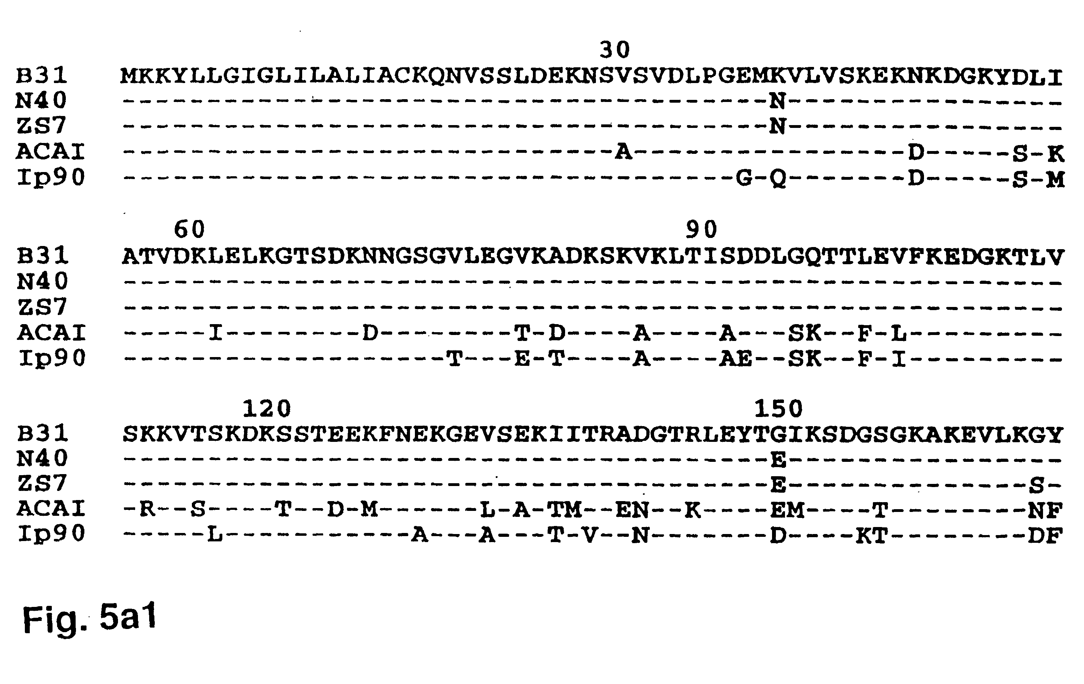

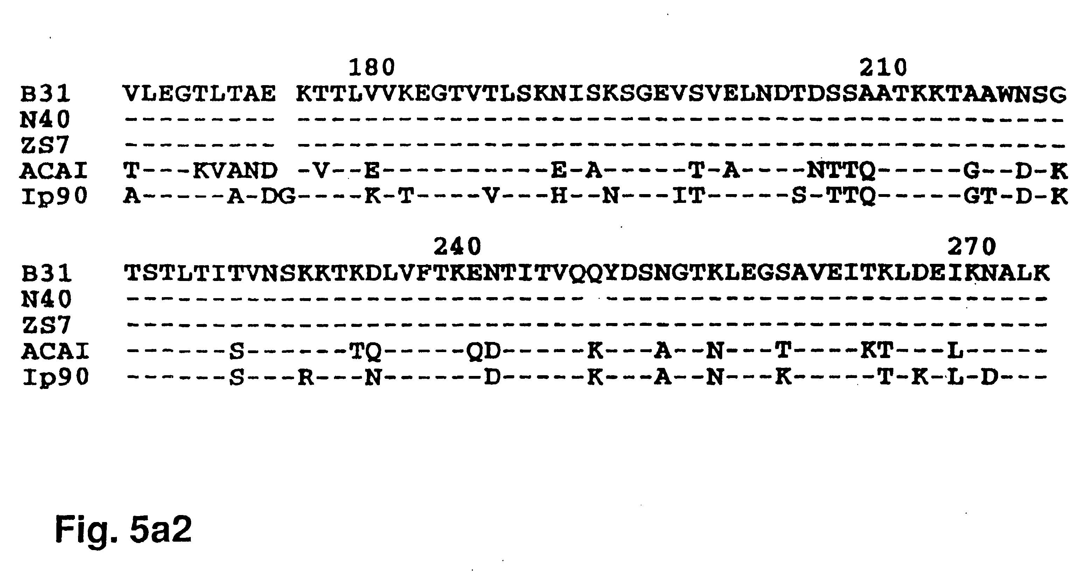

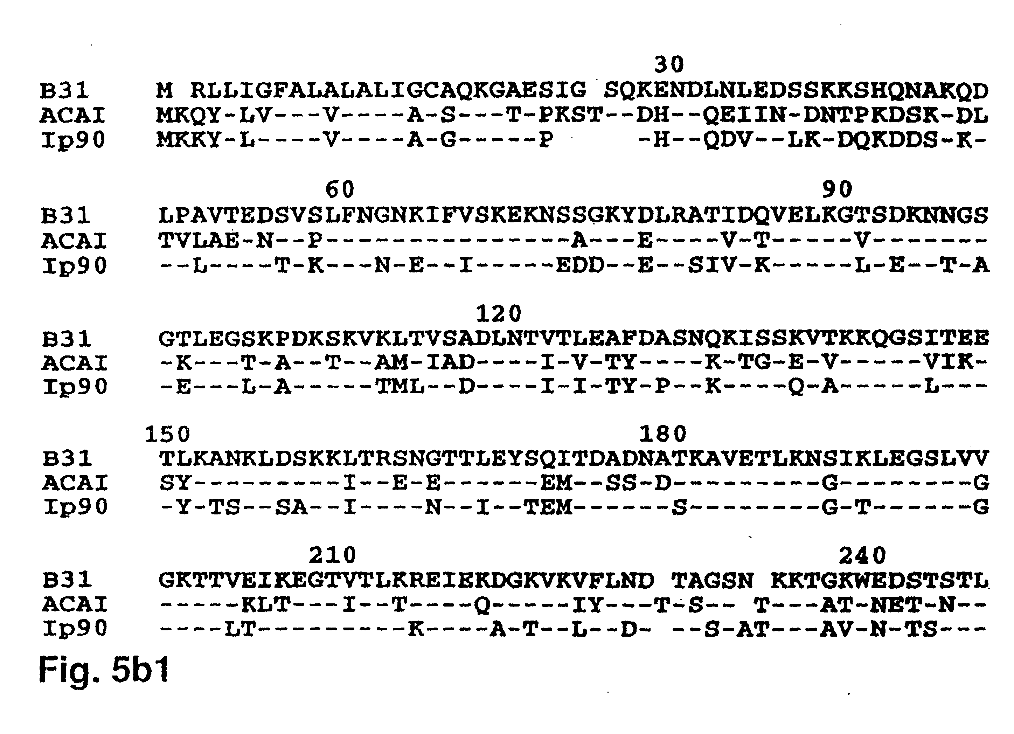

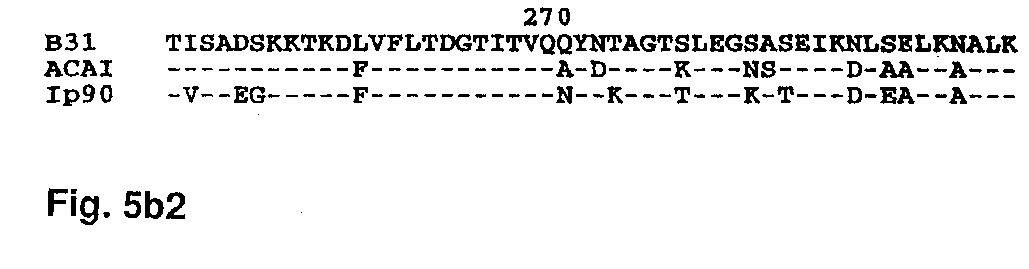

Another type of organism which may be used to express OspA, OspB or a part thereof is a higher eukaryotic organism or cell, including a plant and mammal cell. However, also higher organisms such as animals, e.g. sheep, cattle, goats, pigs, horses and domestic animals, including cats and dogs, are contemplated to be useful as host organisms for the production of OspA or a part thereof. When a higher organism, e.g. an animal, is employed for the production of OspA or a part thereof, conventional transgenic techniques may be employed. These techniques comprise inserting the DNA fragment shown in Fig. 5 or one or more parts thereof into the genome of the animal in such a position that OspA or part thereof is expressed together with a polypeptide which is inherently expressed by the animal, preferably a polypeptide which is easily recovered from the animal, e.g. a polypeptide which is secreted by the animal, such as a milk protein or the like. Alternatively, the DNA fragment of the invention could be inserted into the genome of the animal in a position allowing the gene product of the expressed DNA sequence to be retained in the animal body so that a substantial steady immunization of the animal takes place.

When a microorganism is used for expressing the DNA fragment of the invention, the cultivation conditions will typically depend on the type of microorganism employed, and the skilled art worker will know which cultivation method to choose and how to optimize this method.

The production of OspA or OspB or a part thereof by recombinant techniques has a number of advantages: it is possible to produce OspA or OspB or part thereof by culturing non-pathogenic organisms or other organisms which do not affect the immunological properties of OspA or OspB or part thereof, and it is possible to produce parts of OspA or OspB which may not be isolated from B. burgdorferi strains. High quantities of OspA or OspB or parts thereof may for instance be obtained by using high copy number vectors for cloning the DNA fragment of the invention or by using a strong promoter to induce a higher level of expression than the expression level obtained with the promoters P1 and P2 present on the DNA fragment of the invention. By use of recombinant DNA techniques for producing OspA or OspB or parts thereof, unlimited amounts of a substantially pure protein or polypeptide which is not "contaminated" with other components which are normally present in B. burgdorferi isolates may be obtained. Thus, it is possible to obtain a substantially pure OspA or OspB protein, i.e. OspA or OspB which is not admixed with other B. burgdorferi proteins which have an adverse effect when present in a vaccine or a diagnostic agent in which the OspA or OspB is an intended constituent. A substantially pure OspA or OspB protein or a polypeptide part thereof has the additional advantage that the exact concentration thereof in a given vaccine preparation is known so that an exact dosage may be administered to the individual to be immunized.

Another aspect of the invention are the non-naturally occurring polypeptides encoded by the DNA-sequences in Fig. 3 (corresponding to SEQ. ID. NOS. 4 and 7) for ACA1 and Ip90. Such polypeptides will appear substantially free of peptides with which their natural counterpart would co-occur. In this respect especially lipidated polypeptides are interesting.

Thus, one embodiment of this aspect of the invention relates to a substantially pure polypeptide comprising an amino acid sequence as shown for ACA1 or Ip90 in Fig. 5 (corresponding to SEQ. ID. NOS. 5, 6, 8 and 9, respectively), an antigenic sub-sequence of said amino acid sequence comprising at least one epitope capable of interacting with immunocompetent cells to elicit an immune response against B. burgdorferi, or a polypeptide having a homology of at least about 70% with said amino acid sequence, preferably a homology of at least about 80%, and in particular a homology of at least about 90%. Examples of such substantially pure polypeptides are the amino acid sequences OspA and OspB as shown for ACA1 and Ip90, respectively (corresponding to SEQ. ID. NOS. 5, 6, 8 and 9, respectively), and antigenic sub-sequences thereof. The subsequences will preferably be between 5 and 100 amino acids in length. The subsequences and homologues are typically prepared by recombinant techniques as described above, modifications compared to the sequences illustrated in Fig. 5 suitably being introduced by any of the DNA-modifying techniques described above, such as mutagenesis. Short synthetic polypeptide sequences according to the invention can also be prepared using DNA made by oligonucleotide synthesis, or they can be produced by solid or liquid phase peptide synthesis.

In the present context, the term "polypeptide" is used in its conventional meaning, i.e. as a sequence of amino acids. The amino acids of the sequence may optionally have been modified, after the preparation of the polypeptide, e.g. by chemical, enzymatic or another type of treatment, which does not amend or destroy the immunological activity of the polypeptide to any substantial extent. The polypeptide may be an entire protein, or a subsequence thereof. Especially interesting polypeptides are amino acid sequences comprising epitopes, i.e. antigenic properties of the polypeptide and being capable of evoking an immune response. The minimum amino acid sequence is one which at least comprises a relevant epitope of the polypeptide.

Especially interesting is a substantially pure polypeptide comprising immunogenic fragments able to elicit an immune response against all 3 B. burgdorferi species I, II and III, preferably against the B. burgdorferi strains B31, ACA1 and Ip90.

The antigenicity or immunogenicity of the polypeptides according to the invention can be assessed by usual immunization experiments, using, e.g., rodents such as mice or rabbits as the immunized animal, or using assays, e.g. ELISA, with polyclonal or monoclonal antibodies raised in advance against B. burgdorferi or an immunogenic B. burgdorferi-related protein or polypeptide.

Methods to obtain specific antibodies against short defined peptides and evaluate their immunogenicity have been described previously. One commonly used strategy is to express the peptide as a fusion protein. There are several advantages to use a fusion system for the production of recombinant peptides. First, heterologous proteins and peptides are often degraded by host proteases; this may be avoided, especially for small peptides, by using a gene fusion expression system. Second, general and efficient purification schemes are established for several fusion partners. The use of a fusion partner as an affinity handle allows rapid recovery of the recombinant peptide. Third, by using different fusion partners, the recombinant product may be localized to different compartments of the host cell or secreted into the culture medium.

There are also several methods described for chemical or enzymatic cleavage of the fusion protein that provide efficient strategies to obtain the desired peptide. Frequently employed fusion systems are the Staphylococcal protein A fusion system and the synthetic ZZ variant which have IgG affinity and have been used for the generation of antibodies against short peptides (Löwenadler et al., 1986 [EMBO J.] 55; 1987 [Gene] 56; 1990 [Eur. J. Immunol.] 57; 1991 [FEMS] 58) the glutathione S-transferase fusion system (Smith and Johnson, 1988 [Gene] 60), the β-galactosidase fusion system (Gray et al., 1982, 54), the trpE fusion system (Yansura, 1990 [Methods Enzym. vol. 185] 61). Several of these systems are commercially available as kits, including vectors, purification components and detailed instructions. In brief, the method to obtain short defined epitopes involves the synthesis of the corresponding oligodeoxynucleotide with appropriate termini to facilitate introduction, in translational frame with the fusion partner, into the desired expression vector.

A substantially pure polypeptide prepared as described above wherein epitopes responsible for evoking autoimmunity in animals, including humans, when administered to the animal have been deleted is a very interesting polypeptide for vaccine purposes.

A particular aspect of the invention relates to synthetic polypeptide fragments at least one amino acid sequence selected from 
LVSKEKNKDGKYDL      (SEQ.ID.NO. 2, residues 41-54),
LVSKEKDKDGKYSL      (SEQ.ID.NO. 5, residues 41-54),
 
KGTSDKNNGSGV (SEQ.ID.NO. 2, residues 64-75),
KGTSDKTNGSGV (SEQ.ID.NO. 5, residues 64-75),
KGTSDKNNGSGT (SEQ.ID.NO. 8, residues 64-75),
 
LEGVKADKSKVKL (SEQ.ID.NO. 2, residues 76-88),
LEGTKDDKSKAKL (SEQ.ID.NO. 5, residues 76-88),
LEGEKTDKSKAKL (SEQ.ID.NO. 8, residues 76-88),
 
KKVTSKDKSSTEEK      (SEQ.ID.NO. 2, residues 11-125),
RKVSSKDKTSTDEM      (SEQ.ID.NO. 5, residues 112-125),
KKVTLKDKSSTEEK      (SEQ.ID.NO. 8, residues 112-125),
 
KKTKDLVFTKEN (SEQ.ID.NO. 2, residues 230-241),
KKTTQLVFTKQD (SEQ.ID.NO. 5, residues 230-241),
RKTKNLVFTKED (SEQ.ID.NO. 8, residues 231-242),
 
QYDSNGTKLEGS (SEQ.ID.NO. 2, residues 247-258),
KYDSAGTNLEGT (SEQ.ID.NO. 5, residues 247-258),
KYDSAGTNLEGK (SEQ.ID.NO. 8, residues 248-259),
 
AVEITKLDEIKNALK (SEQ.ID.NO. 2, residues 259-273),
AVEIKTLDELKNALK (SEQ.ID.NO. 5, residues 259-273),
AVEITTLKELKDALK (SEQ.ID.NO. 8, residues 260-274),
 
DLNLEDSSKKSHQNAK    (SEQ.ID.NO. 3, residues 31-46)
DQEIINSDNTPKDSKK    (SEQ.ID.NO. 6, residues 33-48)
DQDVEDLKKDQKDDSK    (SEQ.ID.NO. 9, residues 28-43)
 
KIFVSKEKNSSGK (SEQ.ID.NO. 3, residues 64-76),
KIFVSKEKNSAGK (SEQ.ID.NO. 6, residues 66-78),
EIFISKEKNEDDK (SEQ.ID.NO. 9, residues 61-73),
 
KPDKSKVKLTVSAD (SEQ.ID.NO. 3, residues 105-118),
KADKTKVAMTIADD (SEQ.ID.NO. 6, residues 107-120),
KADKSKVTMLVSDD (SEQ.ID.NO. 9, residues 102-115),
 
KKTGKWEDSTSTL (SEQ.ID.NO. 3, residues 234-246),
KKTATWNETTNTL (SEQ.ID.NO. 6, residues 237-249),
KKTAVWNDTSSTL (SEQ.ID.NO. 9, residues 232-244),
 
KNLSELKNALK (SEQ.ID.NO. 3, residues 286-296),
KDLAALKAALK (SEQ.ID.NO. 6, residues 289-299), and
KDLEALKAALK (SEQ.ID.NO. 9, residues 284-294),
 and polypeptide fragments comprising sequences which are at least 70% homologous, preferably at least 80% homologous, and in particular at least 90% homologous, with any of the above sequences and capable of interacting with immunocompetent cells to elicit an immune response against B. burgdorferi, said polypeptide fragment or fragments being smaller in size than naturally occurring OspA or OspB. As mentioned above, computer analysis has indicated that these sequences are of particular value as epitopes. The above sequences comprise sequences derived from the OspA and OspB proteins from B31 in addition to the proteins from ACA1 and Ip90, the first one in each group of two or three sequences being derived from B31, the second from ACA1, and the third from Ip90 (in the case of the first group, there is complete identity between the sequence (the second sequence of the group) derived from ACA1 and the corresponding sequence derived from Ip90).

The abbreviations of the amino acids used herein should be interpreted as follows:

| | Three-letter | One letter |
|---|---|---|
| Amino acid symbol | abbreviation | |
| Alanine | Ala | A |
| Arginine | Arg | R |
| Asparagine | Asn | N |
| Aspartic acid | Asp | D |
| Asparagine or aspartic acid | Asx | B |
| Cysteine | Cys | C |
| Glutamine | Gln | Q |
| Glutamic acid | Glu | E |
| Glutamine or glutamic acid | Glx | Z |
| Glycine | Gly | G |
| Histidine | His | H |
| Isoleucine | Ile | I |
| Leucine | Leu | L |
| Lysine | Lys | K |
| Methionine | Met | M |
| Phenylalanine | Phe | F |
| Proline | Pro | P |
| Serine | Ser | S |
| Threonine | Thr | T |
| Tryptophan | Trp | W |
| Tyrosine | Tyr | Y |
| Valine | Val | V |

A further aspect of the present invention is an antigenic composition comprising, as an antigenic component, a polypeptide encoded by the DNA fragment as defined above or a polypeptide as defined above.

The antigenic composition may comprise a combination of polypeptides encoded from at least two of the OspA genes from B31, ACA1, and Ip90, and/or a combination of polypeptides encoded from at least two of the OspB genes from B31, ACA1, and Ip90, such as a combination of polypeptides encoded from all three of the OspA genes from the three strains mentioned, optionally combined with polypeptides encoded by one, two, or three of the OspB genes from the three strains in question. Such a composition may be useful in generating an immune response against B. burgdorferi irrespective of the strain of the B. burgdorferi spirochaete.

An example of such a composition is a composition which contains a combination of polypeptides comprising at least one polypeptide fragment selected from 
LVSKEKNKDGKYDL (SEQ.ID.NO. 2, residues 41-54),
KGTSDKNNGSGV (SEQ.ID.NO. 2, residues 64-75),
LEGVKADKSKVKL (SEQ.ID.NO. 2, residues 76-88),
KKVTSKDKSSTEEK (SEQ.ID.NO. 2, residues 11-125),
KKTKDLVFTKEN (SEQ.ID.NO. 2, residues 230-241),
QYDSNGTKLEGS (SEQ.ID.NO. 2, residues 247-258),
AVEITKLDEIKNALK (SEQ.ID.NO. 2, residues 259-273),
DLNLEDSSKKSHQNAK (SEQ.ID.NO. 3, residues 31-46),
KIFVSKEKNSSGK (SEQ.ID.NO. 3, residues 64-76),
KPDKSKVKLTVSAD (SEQ.ID.NO. 3, residues 105-118),
KKTGKWEDSTSTL (SEQ.ID.NO. 3, residues 234-246),
KNLSELKNALK (SEQ.ID.NO. 3, residues 286-296)
 at least one polypeptide fragment selected from 
LVSKEKDKDGKYSL (SEQ.ID.NO. 5, residues 41-54),
KGTSDKTNGSGV (SEQ.ID.NO. 5, residues 64-75),
LEGTKDDKSKAKL (SEQ.ID.NO. 5, residues 76-88),
RKVSSKDKTSTDEM (SEQ.ID.NO. 5, residues 112-125),
KKTTQLVFTKQD (SEQ.ID.NO. 5, residues 230-241),
KYDSAGTNLEGT (SEQ.ID.NO. 5, residues 247-258),
 
AVEIKTLDELKNALK (SEQ.ID.NO. 5, residues 259-273),
DQEIINSDNTPKDSKK (SEQ.ID.NO. 6, residues 33-48),
KIFVSKEKNSAGK (SEQ.ID.NO. 6, residues 66-78),
KADKTKVAMTIADD (SEQ.ID.NO. 6, residues 107-120),
KKTATWNETTNTL (SEQ.ID.NO. 6, residues 237-249),
KDLAALKAALK (SEQ.ID.NO. 6, residues 289-299),
 and at least one polypeptide fragment selected from 
KGTSDKNNGSGT (SEQ.ID.NO. 8, residues 64-75),
LEGEKTDKSKAKL (SEQ.ID.NO. 8, residues 76-88),
KKVTLKDKSSTEEK (SEQ.ID.NO. 8, residues 112-125),
RKTKNLVFTKED (SEQ.ID.NO. 8, residues 231-242),
KYDSAGTNLEGK (SEQ.ID.NO. 8, residues 248-259),
AVEITTLKELKDALK (SEQ.ID.NO. 8, residues 260-274),
DQDVEDLKKDQKDDSK (SEQ.ID.NO. 9, residues 28-43),
EIFISKEKNEDDK (SEQ.ID.NO. 9, residues 61-73),
KADKSKVTMLVSDD (SEQ.ID.NO. 9, residues 102-115),
KKTAVWNDTSSTL (SEQ.ID.NO. 9, residues 232-244),
KDLEALKAALK (SEQ.ID.NO. 9, residues 284-294),
 or polypeptide fragments comprising sequences which are at least 70% homologous, preferably at least 80% homologous, and in particular at least 90% homologous, with any of the above sequences, the combination being capable of interacting with immunocompetent cells to elicit an immune response against B. burgdorferi of any of the strains B31, ACA1, and Ip90, said polypeptide fragment or fragments being smaller in size than naturally occurring OspA or OspB said polypeptide fragments being smaller in size than naturally occurring OspA or OspB, the size here and in the above-discussed contexts typically corresponding to only the amino acid sequences of the sizes as shown or of the same order of sizes.

While the strategy of the above-mentioned composition is to provide an epitope characteristic of each of the strains of B. burgdorferi, another important strategy made possible by the present invention is an antigenic composition comprising a polypeptide fragment selected from 
LVSKEKNKDGKYDL (SEQ.ID.NO. 2, residues 41-54),
LVSKEKDKDGKYSL (SEQ.ID.NO. 5, residues 41-54),
 
KGTSDKNNGSGV (SEQ.ID.NO. 2, residues 64-75),
KGTSDKTNGSGV (SEQ.ID.NO. 5, residues 64-75), and
KGTSDKNNGSGT (SEQ.ID.NO. 8, residues 64-75),
 or polypeptide fragments comprising sequences which are at least 70% homologous, preferably at least 80% homologous, and in particular at least 90% homologous, with any of the above sequences and capable of interacting with immunocompetent cells to elicit an immune response against B. burgdorferi of any of the strains B31, ACA1, and Ip90, said polypeptide fragment or fragments being smaller in size than naturally occurring OspA or OspB, such as discussed above.

As will appear from Fig. 5, there is a very high degree of identity/homology between the sequences shown immediately above in the three strains, and also other regions can be easily identified where there is a high degree of homology, those regions where there is a high degree of homology, e.g. a homology of least 70%, preferably of at least 80%, and in particular at least 90%, and where, at the same time, the homologous polypeptides are immunogenic, such as can be assessed as described above, being candidates for providing single polypeptides which are capable of eliciting a strain-independent immune response against B. burgdorferi.

The capability of evoking an immune response against OspA or OspB, and possibly also against immunogenic determinants thereof, is known as described above to be greater if the polypeptide is lipidated. It is therefore also an aspect of the invention that the polypeptides in an antigenic composition for immunizing purposes comprises lipidated polypeptides, especially if the composition is to be used for priming an immune response. In cases where the antigenic composition is to be used for boosting a previously primed immune response non-lipidated polypeptides may be used as well.

A further aspect of the invention is a vaccine for immunizing mammals, including humans, against Lyme disease, the vaccine comprising an antigenic composition as discussed above and optionally an immunogenically acceptable carrier or vehicle.

The carrier or vehicle may be selected from macromolecular carriers such as a polymer, e.g. a polysaccharide or a polypeptide. In addition to the carrier or vehicle, the vaccine may contain an adjuvant, such as Freund's complete or incomplete adjuvant, aluminum hydroxide, a saponin, a muramyl dipeptide, an iscome and an oil, such as a vegetable oil, e.g. peanut oil, or a mineral oil, e.g. silicone oil.

In the vaccine, the immunogenic component(s) may be coupled to a carrier, in particular a macromolecular carrier. The carrier is usually a polymer to which the immunogenic component(s) is/are bound by hydrophobic non-covalent interaction, such as a plastic, e.g. polystyrene, or a polymer to which the immunogenic component(s) is/are covalently bound, such as a polysaccharide, or a polypeptide, e.g. bovine serum albumin, ovalbumin or keyhole limpet hemocyanin. The carrier should preferably be non-toxic and non-allergenic. The immunogenic component(s) may be multivalently coupled to the macromolecular carrier as this provides an increased immunogenicity of the vaccine preparation. It is also contemplated that the immunogenic component(s) may be presented in multivalent form by polymerizing the immunogenic component(s) with itself.

In this regard, it may prove advantageous to couple the immunogenic component to the carrier together with one or more immunologically active molecules obtained from organisms other than B. burgdorferi so as to obtain a vaccine comprising a variety of different immunogenic determinants, being a cocktail vaccine, which may be employed for the immunization against diseases caused by other organisms, e.g. organisms responsible for relapsing fever or syphilis.

In another embodiment, a mixture of two or more single vaccines may be employed.

It is known that antibodies raised against B. burgdorferi or parts thereof evoking an immune response have a rather short lifetime in sera of animals and humans. Thus, a suitable strategy for immunizing animals and humans against Lyme disease is to periodically administer the vaccine described above to individuals subjected to contact with ticks bearing B. burgdorferi, i.e. boosting at regular intervals. It is contemplated that vaccination once a year such as in the springtime will provide a suitable protection of individuals in risk of B. burgdorferi infection. A suitable dose of immunogenic components for such a vaccination is 5-500 µg. However, also more irregular immunizations may be advantageous, and any immunization route which may be contemplated or shown to produce an appropriate immune response can be employed in accordance with the principle of the present invention. Suitable administration forms of the vaccine of the invention are oral administration forms, e.g. tablets, granules or capsules, subcutaneous, intracutaneous or intramuscular administration forms or forms suitable for nasal or rectal administration.

While the vaccines and compositions discussed above will elicit a humoral immune response, it is possible additionally to evoke a cellular response by incorporating, together with the polypeptide, the carrier, and the optional adjuvant, a peptide expressing a T-cell epitope. If desired, such a T-cell epitope peptide can be bonded to the above-discussed polypeptide by peptide bonds and can be encoded from the same DNA fragment which encodes the above-discussed polypeptide, the DNA fragment having been supplemented with a nucleotide sequence coding for the T-cell epitope peptide.

One interesting method for selecting or developing epitopes eliciting immune responses to B. burgdorferi is to utilize a selection pressure invoked by incubation with monoclonal antibodies and investigating sequences of the OspA and OspB proteins of the surviving variants of B. burgdorferi. This method is illustrated in Example 6. Epitopes detected by this method is also understood to be within the scope of the present invention.

As stated above, recombinant DNA technologies are useful for the preparation of diagnostic reagents and vaccines. Routine methods for vaccine production involve risks of obtaining unwanted side effects, e.g. due to the vaccine containing unwanted (or even unidentified) contaminants. An alternative approach to the production of new vaccines involves the insertion of one or more DNA sequences constituting one or more parts of the DNA sequence shown in Fig. 5 or parts thereof into a virus genome, e.g. into a retrovirus, vaccinia virus or Epstein-Barr virus genome, to produce a polyvalent vaccine. An especially interesting virus for the present purpose is vaccinia. Also, synthetic polypeptides which have been prepared by conventional methods, e.g. by solid or liquid phase peptide synthesis, are suitable for vaccines.

In a further aspect, the present invention relates to a non-pathogenic microorganism which carries and is capable of expressing an inserted nucleotide sequence which is the nucleotide sequence shown in Fig. 5 or part thereof for use as a live vaccine for the immunization of an animal against Lyme disease. For instance, the use of a live vaccine might be advantageous since it is presumed that vaccines based on living organisms show an excellent immunogenicity, and it is also contemplated that the use of a live vaccine will confer a life-long immunity against Lyme disease so that repeated vaccination will not be needed.

In a particularly advantageous embodiment of the live vaccine of the invention, the DNA fragment of the invention is expressed on the outer surface of the host microorganism. This provides a favourable presentation of the immunologically active part(s) of OspA recognized by the immune defense mechanisms of the animal to which the live vaccine is administered, thus provoking an appropriate immune response. One way of providing the expression of OspA or immunologically active part(s) thereof (the epitopes) on the cell surface is to fuse the DNA fragment of the invention to another nucleotide sequence encoding a surface protein or a subsequence thereof (e.g. a signal peptide) which cause the B. burgdorferi epitopes to be expressed on the outer surface of the host cell, optionally as a fused polypeptide. Examples of useful surface proteins are adhesins, fimbrial proteins, or other extracellular proteins.

The microorganism used for live vaccines should be a non-pathogenic microorganism, e.g. a non-pathogenic E. coli, which may be able to establish itself in the animal body. A microorganism which may prove especially useful as a live vaccine may be the B. burgdorferi in itself, which as explained above inherently expresses OspA on the surface of the cell. The use of B. burgdorferi for a live vaccine requires, however, that the B. burgdorferi has been altered so as to not cause any illness when used as a live vaccine. This alteration or modification may be carried out in any suitable manner, e.g. by mutagenization, chemical, enzymatic or heat treatment, or by another equivalent treatment resulting in an attenuated B. burgdorferi cell.

In order to increase the production of lipidated recombinant Borrelia burgdorferi outer surface protein, OspA, in E. coli, a specially designed expression system has been developed.

The results obtained by using the original OspA signal peptide in different E. coli expression vectors have demonstrated that the signal peptide cleavage site is recognized. Furthermore, the processed OspA molecules are also lipidated, demonstrated by ³H-palmitic acid labelling and protein characterization. However, the level of production is relatively low.

To improve productivity and facilitate signal peptide cleavage and lipidation, the sequence encoding the full-length OspA protein is combined with the amino-terminal cysteine residue with an E. coli derived signal sequence. Since it is well known that the signal peptidase II recognizes certain signal peptides and is also responsible for lipidation of pre-proteins harbouring such a sequence, it is reasonable to assume the advantage of using a homologous sequence. The resulting recombinant open reading frame is then to be inserted under control of a strong promoter.

A further aspect of the present invention is a method of producing a polypeptide as defined above comprising
- inserting a DNA fragment as defined above in a vector which is able to replicate in a specific host cell,
- introducing the resulting recombinant vector into the specific host cell,
- growing the host cell under appropriate culture conditions for expression of the polypeptide, and
- recovering the polypeptide,
optionally followed by purification.

Preferably, the host cell is capable of expressing lipidated polypeptides, such as is the case when the host cell is E. coli.

### Hybridization of DNA

DNA, e.g. present on nitrocellulose filters, are wetted in 2 x SSC [1 x SSC: 0.15 M NaCl, 0.0015 M Na₃-citrate, pH 7.0] and placed in a heat-sealed plastic bag with prewarmed 67°C) prehybridization solution. Prehybridization takes place for 2 h at 67°C, the bag being gently shaken. The solution is exchanged with prewarmed (67°C) hybridization solution, the radioactive probe is added and hybridization is carried out at 67°C for 18 h. The bag is gently shaken to ensure constant movement of the liquid over the nitrocellulose filters. After hybridization, a washing procedure is carried out.

The radioactive probe is prepared by use of known methods, e.g. as described by Sambrook et al., on the basis of the DNA sequence shown in Sequence Listing 1 or a part thereof, especially a coding part such as the nucleotides corresponding to amino acids 1-210 or an effective subsequence of the DNA sequence as defined above.

The prehybridization and hybridization solutions used are: 10 x Denhardt's, 4 x SSC, 0.1% SDS, 10 µg/ml polyA, 50 µg/ml of denatured DNA to be analysed and the denatured (heat) radioactive probe. The filters are washed in prewarmed (67°C) solutions: 10 x Denhardt, 2 x SSC, 0.1% SDS for 2 x 15 min. and 1 x SSC, 0.1% SDS for 4 x 15 min. The filters are air-dried and covered with Vita-Wrap, and X-ray film is exposed to the filters for 3 h to 3 weeks with and without intensifying screens.

### EXAMPLE 1

### Isolation and sequence analysis of the OspA and OspB genes

### Bacterial strains

The Borrelia burgdorferi strains used were the American reference strain B31 (ATCC 35210), the Swedish isolate ACAI isolated from a patient with acrodermatitis chronicum migrans (1), and the Ip90 strain which is isolated from I. persulcatus from the Soviet union and was kindly provided by E. I. Korenberg and V. N. Kryuchechnikov of the Gamaleya Institute, Moscow (27).

### Media and culturing conditions

The B. burgdorferi strains were cultivated in BSK II medium as previously described (2).

The Escherichia coli strains DH5α (BRL, Gaithersburg, Md. USA) and Y1090 (24) were used for propagation of recombinant plasmids and for growth of λgt11 phage gene library, respectively.

### Construction and screening of λ-gt11 and pUC18 plasmid B. burgdorferi gene libraries

B. burgdorferi strains were cultured in 400 ml modified BSKII-medium (2) at 34°C and harvested at late mid-log phase. The DNA was extracted and purified as previously described (23). The ACAI DNA was partially digested by Sau3AI and fragments 4-8 kb in size were isolated from a 0.7% agarose gel. The fragments were then ligated into BamHI-cut λ-gt11 arms and packaged into phage heads as described by the vendor (SDS-Promega, Falkenberg, Sweden). The λ-phages were propagated in E. coli Y 1090 and screening, by DNA hybridization, of the λ-library was according to standard methods (24). The oligonucleotides used for screening, J1, J2, and J3, were synthesized from the previously published B. burgdorferi B31 osp-operon nucleotide sequence (14). All nucleotides used for screening of phage and plasmid libraries and primers for nucleotide sequencing are shown in Table 1. The oligonucleotides were end labelled with ³²P-γ-dATP, as previously described (31), and purified on a Sephadex G-50 (Pharmacia, Uppsala, Sweden) spin column. The hybridization was performed at 37°C, i.e. medium stringency, as some differences in the nucleotide sequence between the different strains could be expected. The phage DNA was extracted as previously described (28). Purified λ-DNA was further subcloned into the EcoR1 site of pUC18 according to standard methods (30).

A plasmid gene library of B. burgdorferi Ip90 DNA was constructed by partial Hind III digestion of total DNA, in which 1 u enzyme was incubated with 100 ng DNA at 37°C for 15 min. The reaction was terminated by a phenol:chloroform (1:1) extraction. After ethanol precipitation, the fragments were ligated with 30 ng HindIII digested pUC18 plasmid DNA at 16°C for 4 h. The plasmid were transformed into competent E. coli DH5α cells. An additional plasmid gene library was constructed by complete EcoRI digestion of IP90 DNA and cloning into the EcoRI site of the plasmid pUC18.

The B. burgdorferi Ip90 plasmid gene library was screened with a 259 bp osp-fragment. This ospA fragment was obtained by PCR amplification of a DNA segment located at the end of the ospA gene, using the J1 and K1 oligonucletides as primers (Table 1). This fragment corresponds to a fragment between nucleotide positions 529 to 788 of the B. burgdorferi B31 ospA gene. The conditions for the PCR amplification were as follows; 16.6 mM (NH₄)SO₄, 67 mM Tris-HCl (pH 8.8 at 25°C), 6.7 mM MgCl₂, 10 mM 2-mercaptoethanol, 200 µM each of dATP, dGTP, dCTP, and dTTP, 170 µg per ml bovine serum albumin, 5 pmol of each primer, about 10 pg total Ip90 DNA and 1 u of BioLabs Taq polymerase. Reactions were performed in a volume of 50 Ml, overlaid with 40 Ml of mineral oil. During the first 5 cycles, denaturation was done at 94°C for 2 min, annealing at 45°C for 1 min and elongation at 72°C for 1 min. In the next 30 cycles, the denaturation time was shortened to 39 sec and annealing temperature raised to 55°C, otherwise the conditions were the same. The PCR fragments was labelled with ³²P-α-dATP using an oligo-labelling kit (Amersham, Buckinghamshire, UK) and used to screen the HindIII library for osp-gene containing clones. The screening was performed as described (30), and the hybridization was performed at high stringency (60°C).

### Nucleotide sequence analysis

Deletion libraries in one direction were constructed for the full length clone of ACAI and the shorter or the Ip90 clones as described earlier (21). Mini plasmid preparations were either performed according to the boiling method (30) or by a CsCl method previously described (39). Nucleotide sequencing was performed using the dideoxy chain-termination method described by Sanger et al. with the use of Pharmacia ^{T7}Sequencing™ kit (Pharmacia, Uppsala, Sweden). The complete sequence for both strands was determined. From the nucleotide sequences obtained, internal primers were synthesized to enable sequencing of the other strand plus the beginning of the Ip90 osp-operon. The sequences obtained from the DNA deletion sequencing method were assembled using the GENEUS (20) software for VAX computers (Digital Equipment Corporation). Additional nucleotide sequence analyses were performed using the University of Wisconsin GCG Sequence Analysis software for the VAX computer, and PC-Gene (Genofit) for the personal computers.

### In vitro transcription analysis

Transcriptional analysis was performed on total mRNA isolated form B. burgdorferi B31 using a method previously described (31). The isolated and purified mRNA was subjected to primer extension in an in vitro reaction using Avian myeloblastosis virus reverse transcriptase (Lief Sciences, Fla. USA) as described previously with minor modifications (13, 17, 48). The primer extension reaction with RNA was carried out with [α-³²P]-dATP. The primer used in the primer extension reaction was the reverse complement of nucleotides 128 to 160 in Figure (3). Only full-length mRNA polymer was synthesized, the size of the transcript was compared with a regular Sanger dideoxy DNA sequence on plasmid pTHR44 obtained when using the same oligonucleotide primer. In the DNA sequencing reactions [α-³⁵S]-dATP was used.

### EXAMPLE 2

### Cloning and nucleotide sequence analysis of osp-operons from B. burgdorferi isolates ACAI and Ip90

### Sequencing analysis

The osp-operons from the Swedish B. burgdorferi strain ACAI was isolated from a λgt11 library. The isolation of the ACAI osp-operon was performed by screening with a mixture of three oligonucleotides, J1, J2 and J3, which were synthesized from the nucleotide sequence of the previously published B. burgdorferi B31 osp-operon. One positive λ-clone containing the ACAI ospA and ospB genes was isolated and characterized by restriction endonuclease mapping. The isolated osp operon was subcloned into pUC18 and the genetic organization of the genes was confirmed with the same three oligonucleotides, J1, J2, and J3. The osp-operon of strain Ip90 was cloned for a pUC18 plasmid library using Hind III, partially digested, total Ip90 B. burgdorferi DNA. One positive clone containing almost the entire Ip90 osp-operon except the first 175 bp of ospA was obtained. An oligonucleotide, J4, was constructed from the start of this Hind III clone. This oligonucleotide, J4, was used to pick up a clone containing the whole Ip90 osp-operon from a pUC18 plasmid library of completely EcoRI digested Ip90 DNA.

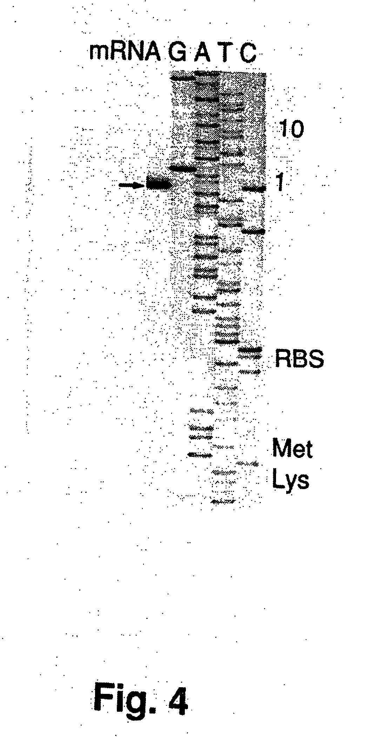

The osp-operons were sequenced and their nucleotide sequences of the osp-operons of strains ACAI and Ip90 are shown in Figure 3. The obtained nucleotide sequences are, in the Figure, aligned and compared to the previously published nucleotide sequences of the ospA and ospB genes of strain B31 (14) and the ospA sequences from the strains Z57 (45) and N40 (19). The percentage DNA identity of the respective ospA and ospB genes are shown in Table 2 and 3. The results of the nucleotide sequence comparison revealed that the ospA genes from strains ACAI and Ip90 exhibited a sequence identity of 85% with the ospA genes of strain B31. When compared to each other, the ospA genes of ACAI and Ip90 also showed an 85% similarity. In contrast, the two previously published ospA sequences from strains Z57 and N40 were almost identical to the ospA sequence of strain B31 (>99%). The ospB sequences of strain ACAI and Ip90 were 79% identical to the ospB gene of strain B31 and 81% identical compared to each other. Further analysis of the osp-genes of strain ACAI and Ip90 revealed that ospA and ospB genes are organized in one operon, separated by just a few basepairs. The osp-operons in these two strains are also preceded by a control region consisting of a σ-70 promotor and a Shine and Dalgarno ribosomal binding site, as depicted in figure 3 (37). In order to determine the exact transcriptional start point of the osp-operon, an in vitro primer extension was performed on isolated total messenger RNA from B. burgdorferi B31 (Figure 4). The in vitro transcription analysis identified the transcriptional start site as the G at position +1 (Figure 3). This transcriptional start site is situated 36 bp upstream of the AUG translational start codon.

### EXAMPLE 3

### Sequence analysis of the translated OspA and OspB proteins of strains ACAI and Ip90.

The translated products of the ospA genes of ACA1 and Ip90 were compared with the deduced translation products of strains B31, ZS7, and N40. The comparison of the different OspA proteins in an optimal alignment are shown in Figure 5A. The deduced OspA protein for ACA1 is 273 amino acids long with a theoretical molecular weight of 29,629 and for Ip90 it is 274 amino acids long predicting a protein with a molecular weight 29,673. The three different ospB gene products are compared in Figure 5B. The deduced OspB proteins have a molecular weight of 32,432 coded by 299 amino acids for ACA1, and 32,105 coded by 294 amino acids for Ip90. From the amino acid sequence comparison of the OspA and OspB proteins it is evident that the start of the OspA is very conserved between the different strains, while the middle and the C-terminal parts of the proteins show a higher degree of variation. In the OspB protein the same overall variability in the sequence is seen. From the deduced amino acid sequence of the OspA and OspB proteins of B. burgdorferi strain B31 the sequence similarity with a prokaryotic lipoproteins was shown (14). The deduced OspA and OspB proteins of B. burgdorferi strains ACA1 and Ip90 also contain the typical consensus tetrapeptide (LXYC) in their peptides. The possible signal peptidase II recognition sites of the OspA and OspB proteins are indicated in the Figures (5A and 5B).

### EXAMPLE 4

### Extraction of B. burgdorferi proteins, SDS-PAGE, and Western blotting.

The cells were grown in 200 ml at 34°C and harvested in mid-log phase (approximately 2 to 4x10⁸ cells per ml) by centrifugation at 8,000 g for 20 min. The cells were washed twice in PBS-5 mM MgCl₂, and the pellets were resuspended in 2 ml PBS. To prepare soluble proteins, the cells were sonicated 4 times 30 sec in an ice bath by using a Branson Sonifer cell disrupter B15 at setting 3. After centrifugation at 10,000 g for 30 min, the supernatant was collected and the amount of protein was determined by using the Bio-Rad protein assay (Bio-Rad, Munich, Germany).

SDS-PAGE was performed essentially as described before (10) using either 12.5% or 15% acrylamide running gels and 4% acrylamide stacking gels. In each lane 10 to 15 µg protein was added. The gels were either fixed and stained by Coomassie brilliant blue (Sigma chemical, Saint Louis, Mo, USA) or processed for immunoblotting. Molecular weight standards were obtained from Pharmacia (Uppsala, Sweden) and included proteins ranging from 14.4 to 94 kDa. The proteins were transferred to immobilon filters (Millipore Corporation, Bedford, Ca, USA) by electroblotting at 0.8 mA over cm² for 45 min. The non-specific binding on the filters was blocked by incubation with 5% milk powder in PBS over night. The filters were then incubated with antibodies (1:20 or 1:25 dilution in 2.5% milk powder in PBS), washed 3 times 5 min in PBS-0.05% Tween 20 and then incubated with an appropriate peroxidase labeled monoclonal antibody, Mab (1:500 dilution in 2.5% milk/PBS) for 1 hour. Bound antibodies were then visualized by adding 5-bromo-4-chloro-3-indolylphosphate as peroxidase substrate. All incubations were performed during continuous shaking. The Mab's used were the anti-OspA antibodies H5332 (10) and the H3TS (7) and the anti-OspB antibody H6831 (9). A polyclonal antisera raised against whole cell lysates form the Swedish B. burgdorferi tick isolate G152 (44), a gift from Mats Karlsson, Danderyd hospital, Stockholm, Sweden, was used in Western blotting to show the presence of B. burgdorferi major outer surface proteins.

### EXAMPLE 5

### Biochemical and immunochemical characterization of the OspA and OspB proteins of B. burgdorferi strains ACA1 and Ip90.

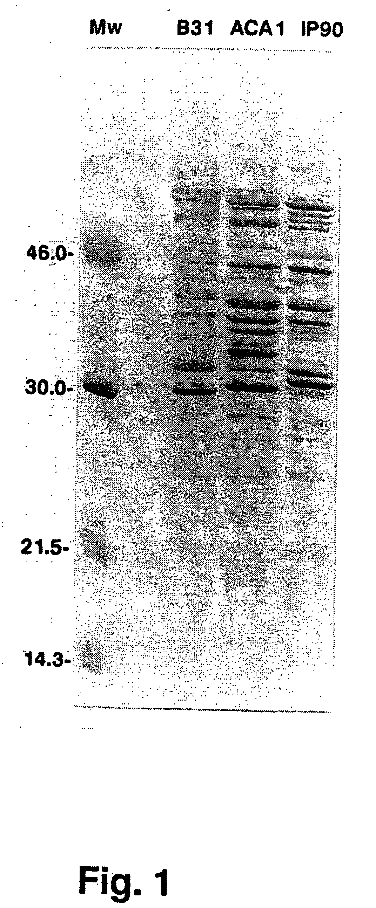

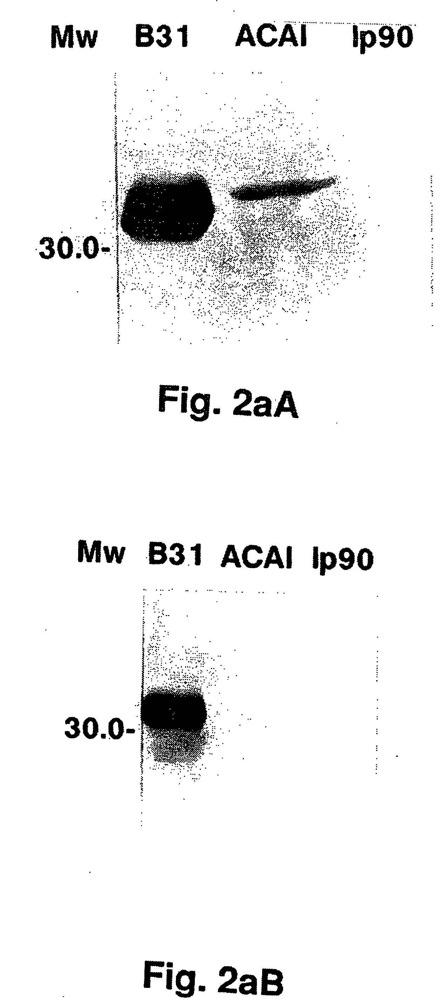

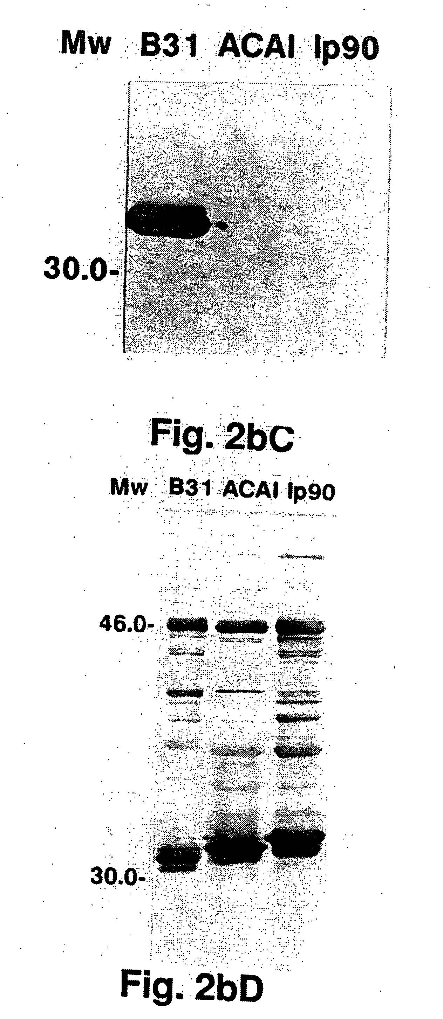

Whole cell protein extracts prepared form B. burgdorferi strains B31, ACA1 and Ip90 separated on a 12,5% SDS-PAGE are shown in Figure 1. These three different B. burgdorferi isolates were obtained from different geographical locations. The three strains show different apparent molecular weights for both the major outer surface proteins OspA and OspB. This result was also found earlier, when comparing the strains B31 and ACA1 (8). The molecular weights of the respective OspA and OspB proteins as determined from the SDS-PAGE are as follows: for B31; 31 kD and 34 kD, for ACA1; 32 kD and 36 kD, and for Ip90; 33,5 kD and 34 kD. The Osp proteins from the three different isolates were further characterized by western blot analysis using different monoclonal antibodies directed against the OspA and OspB proteins of B. burgdorferi B31. In a western blot using the OspA specific MAb H5332 (Fig. 2A), a protein extract form strain B31 strongly bound to this antibody, whereas the ACA1 OspA protein only reacted weakly. No protein from strain Ip90 reacted with the MAb H5332. When the OspA specific MAb H3TS (Fig. 2B) or OspB specific MAb H6831 (Fig. 2C) was used only the OspA and OspB proteins of strain B31 reacted, the Osp proteins of ACA1 and Ip90 did not react with these two monoclonal antibodies indicating a variability of the OspA and OspB proteins in these three strains. When using a polyvalent polyclonal antisera raised against whole cell B. burgdorferi (Figure 2D), all three strains reacted with 10 to 15 different proteins. In all strains the strongest hybridization signal was against a protein of the size of the OspA protein. The apparent molecular weights of these presumed OspA proteins are, as calculated from the western blot, 31 kD (B31), 32 kD (ACA1), and 33.5 kD (Ip90), respectively.

### EXAMPLE 6

### Identification of epitopes

### Materials

### B. burgdorferi organism of the strains B31, ACA1 and Ip90

Monoclonal antibodies directed to OspA and OspB, H5332 and H3TS, respectively.

### Methods

Incubating B. burgdorferi in the presence of one of the monoclonal antibodies at 34°C in BSKII broth culture medium, the growth of the spirochates is inhibited. After 1-7 days variants which are not inhibited by the antibody begin to grow. These antibody-restistant organisms have changes in the OspA or OspB protein. They are mutants selected with monoclonal antibodies.

The epitopes against which the growth-inhibiting antibodies are directed will be identified by comparing the deduced amino acid sequence of the original strain with the deduced amino acid sequence of the mutant.

The deduced amino acid of the mutant OspA proteins will be determined by DNA sequencing of amplified DNA from the polymerase chain reaction.

### EXAMPLE 7

### Preparation and evaluation of epitopes

Epitopes are prepared as fusion proteins by using a gene fusion expression system making fusion partners. A fusion partner may be used as an affinity handle which allows rapid recovery of the recombinant peptide. By using different fusion partners, the recombinant product may be localized to different compartments of the host cell or secreted into the culture medium.

The following epitope is prepared LVSKEKNKDGKYDL (SEQ. ID. NO. 2, residues 41-54), by using the following oligodeoxynucleotide sequences: 5'-AATTCGTTAGTATCTAAAGAAAAAA ACAAAGATGGAAAATATGATTGA-3' (SEQ. ID. NO. 14) and 5'-AGCTTC-AATCATATTTTCCA TCTTTGTTTTTTTCTTTAGATACTAACG-3'(SEQ. ID. NO. 15) The oligodeoxynucleotide sequences are synthesized by machine and annealed. The addition of nucleotides at both termini to get EcoRI and HindIII compatible ends, respectively, is to facilitate cloning of the fragment in translational frame into a fusion system expression vector. In this experiment, the commercially available system pEZZ18, based on the synthetic dimer of the Staphylococcal protein A IgG binding domain, ZZ (Löwenadler et al., 1987 [Gene]), under control of the protein A promoter and signal sequence (Pharmacia) is used. The vector plasmid pEZZ18, which contains a multiple cloning site downstream of the ZZ fragment, is digested with EcoRI and HindIII and the 4.6 kb fragment is isolated. The linearized pEZZ18 plasmid element is then ligated with the annealed synthetic oligonucleotides described above. E. coli strain TG2 is transformed, single colonies are isolated, and plasmids are prepared for sequence analysis. Plasmids containing the desired sequence are then transformed into TG2 and DH5α for expression studies. Bacterial cultures are grown in standard LB medium containing 50 µg/ml of ampicillin or carbenicillin. Overnight cultures are harvested by centrifugation and the localization of the recombinant peptide in culture medium, periplasm or cytoplasm is analyzed by fractionation and Western blot experiments. The fraction(s) containing the recombinant fusion protein including the desired peptide is/are then passed over an IgG sepharose, 6FF, column (Pharmacia) for binding of the fusion protein. The column is then extensively washed and the recombinant fusion protein is eluted according to the vendor's instructions (Pharmacia). The column is equilibrated with 2-3 bed volumes of 0.5 M HAc pH 3.4, and with 50 mM Tris-HCl pH 7.6, 150 mM NaCl, 0.05% Tween 20 (TST). The neutral pH is checked before the sample is applied. The sample is applied and the column is washed with 10 bed volumes of TST and bed volumes 5 mM NH₄Ac pH 5.0. The fusion protein is then eluted with 0.5 HAc pH 3.4.

There are options available to localize the fusion partner at either the amino terminal or at the carboxy terminal, or at both. This can be of advantage when specific modifications are desired, for example lipidation of a cysteine localized at the amino terminal.

The recombinant peptide is then generally purified by affinity chromatography, using the fusion partner as a handle (see references above).

Suitable animals are then immunized with the peptide, optionally together with an adjuvant. Sera are collected and evaluated for reactivity against the native protein in an immunoassay, e.g. Western blot or in an in vitro assay, e.g. growth inhibition studies.

### EXAMPLE 8

### Expression of recombinant OspA lipo-protein in Escherichia coli

In order to increase the production of lipidated recombinant Borrelia burgdorferi outer surface protein, OspA, in E. coli, a specially designed expression system has been developed which produces lipidated OspA in E. coli.

### Method

Two PCR primers, SYM 2692 (5'-GCGAATTCGCGGCCGCTGGCTCTGCAGAGCAATCTG-3', SEQ. ID. NO. 16) and SYM 2693 (5'-GCGGATCCGCTAGCAGAGTAGAACCCAGGATTAC-3', SEQ. ID. NO. 17), were synthesized. These primers were used to amplify a fragment containing the hybrid LPP and lac promoter together with the major part of the LPP signal sequence from the plasmid pKEN125, cf. Nakamura et al. 1979 (64). LLP is the gene coding for outer membrane lipoprotein of E. coli (Nakamura et al., 1982) (63). The resulting PCR fragment was digested with PstI and NheI and isolated by agarose electrophoresis.

To amplify the rest of the LPP signal sequence in frame with the 5'-end of the OspA sequence, another PCR was performed. The set of primers used in this reaction was SYM 2695 (5'-GCGAATTCGCTAGCAGGTTGTAAGCAAAATGTTAGCAG-3', SEQ. ID. NO. 18) and SYM 1983 (5'-TCAAGCTTGTCTACTGTTGC-3', SEQ. ID. NO. 19), cf. Nakamura et al. 1979 (64). The amplified fragment was digested with EcoRI and HindIII and isolated as above. This fragment was then ligated with a 645 bp long HindIII and BamHI fragment containing the rest of the OspA encoding sequence and with BamHI and EcoRI digested pUC19, and the resulting plasmid was sequenced and designated pS324.

The plasmid pS324 was then digested with NheI and BamHI, and a 784 bp fragment containing the entire OspA encoding sequence and the sequence encoding the three amino acids from the LPP signal sequence located adjacent to the OspA sequence was isolated by agarose electrophoresis. In the next step, this 784 bp fragment was ligated with the PstI and NheI PCR fragment containing the promoter sequence and the 5'-part of the signal sequence and cloned into PstI and BamHI digested pUC19. This expression construct was designated pS420.

### Expression

To analyze expression directed by this construct, pS420, two E. coli strains were tested, DH5α and TG2.

### DH5α

### Genotype

supE44 ΔlacU169(Φ80lacZΔM15)
hsd R17 recA1 endA1 gyrA96 thi-1 relA1

The Φ80lacZΔM15 permits α-complementation with the amino terminus of β-galactosidase encoded in pUC vectors. DH5α is available from Bethesda Research Laboratories Inc. (Hanahan D. (1983) J. Mol. Biol. 166:557.

### TG 2

### Genotype

supE hsdΔ5 thi Δ(lac-proAB)
Δ(srl-recA)306 Tn10 (tet^{r})
FÛ[traD36 proAB+lac19 lacZΔM15]

A recombination-deficient derivative of TG1. (Sambrook (1989)).

Both strain were obtained from Departement of Microbiology, University of Umeå.

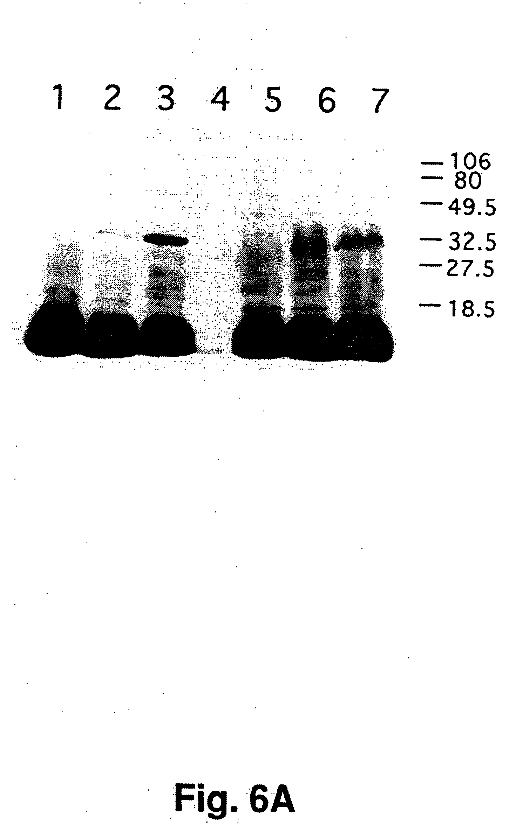

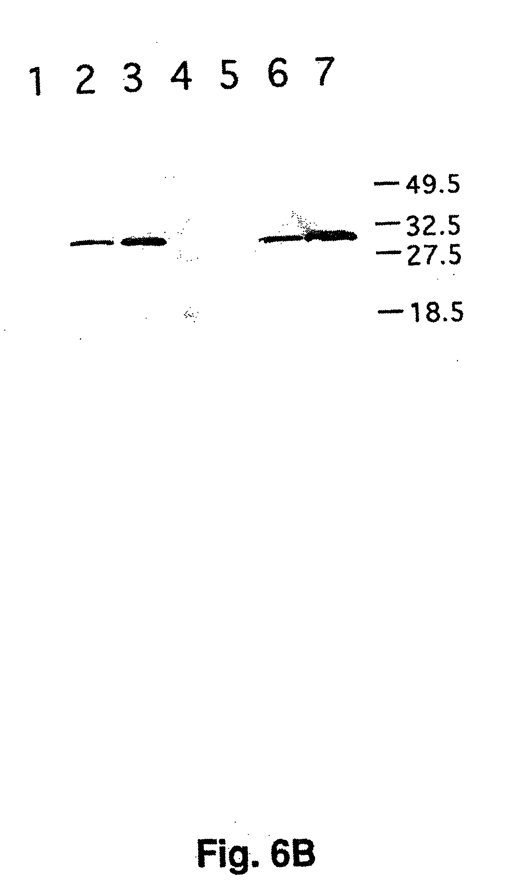

Typical for in vivo labelling studies, the expression experiments were carried out as follows. Liquid cultures, in LB containing 50 µg/ml of carbenicillin, of pS420 transformed DH5α and TG2, were inoculated and incubated at 37°C for approximately 7 hours. From these cultures, 100 µl of culture was inoculated to 1 ml of LB containing 50 µg/ml carbenicillin, 50 µl of [9,10(n)-³H] palmitic acid (Amersham) with a specific activity of 54.6 Ci/mmol and a radioactive concentration of 1 mCi/ml, and 2 mM IPTG. This culture was grown overnight at 37°C. As controls the E. coli host strains without expression vector, and the same strains with the pS151 vector were used. The culture conditions were identical except that carbenicillin was omitted from the non-plasmid containing cultures. pS151 is identical to pTRH44 (Bergström et al., 1989, (13)). Results shown in Fig. 6A and B.

For expression analysis, 1 ml of bacterial culture was harvested and the cells were pelleted by centrifugation. The resulting pellets were dissolved by boiling for 10 minutes in 50 µl of sample buffer (Laemmli, 1970). Samples were analyzed by SDS-PAGE and immunoblotting and autoradiography, respectively. To culture larger volumes, the same procedure was followed except that the radioactive tracer was omitted.

### Conclusion

Analysis by SDS-PAGE and immunoblotting with the OspA monoclonal antibody H5332, in vivo labelling studies with ³H-palmitic acid, and purification of recombinant OspA have demonstrated highly efficient expression of lipidated processed OspA in E. coli.

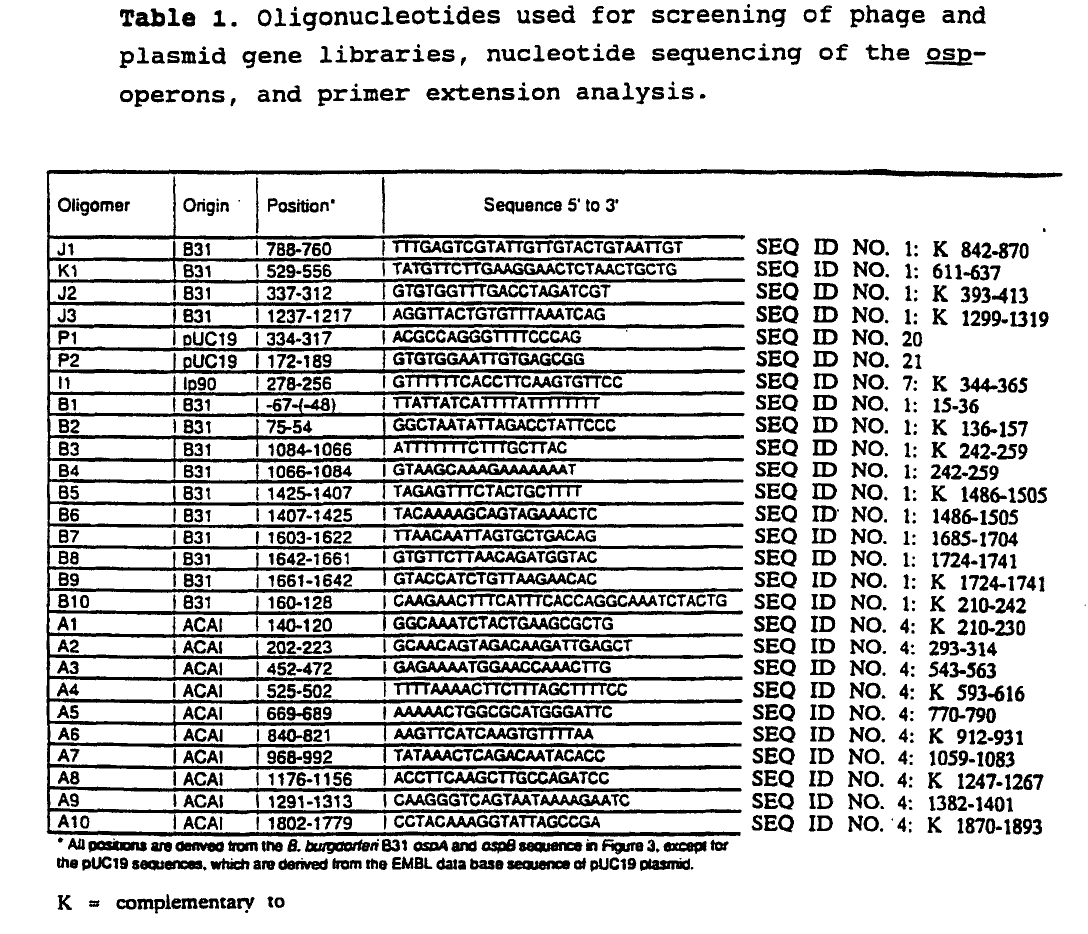

**Table 2.**

| DNA sequence identity (percent) between the ospA genes from different Borrelia burgdorferi isolates. | | | | | |
|---|---|---|---|---|---|
| | B31 | N40 | ZS7 | ACA1 | IP90 |
| B31 | * | | | | |
| N40 | 99.8 | * | | | |
| ZS7 | 99.5 | 99.8 | * | | |
| ACA1 | 85 | 85 | 85 | * | |
| IP90 | 86 | 86 | 85 | 86 | * |

**Table 3.**

| DNA sequence identity (percent) between the ospB genes from different Borrelia burgdorferi isolates. | | | |
|---|---|---|---|
| | B31 | ACA1 | IP90 |
| B31 | * | | |
| ACA1 | 79 | * | |
| IP90 | 79 | 81 | * |

### BRIEF DESCRIPTION OF THE DRAWINGS

**Figure 1** Coomassie blue stained 12.5% SDS-PAGE of whole cell lysates of B. burgdorferi. Lane A: whole cell extract of B. burgdorferi strain B31, lane B: strain ACA1 and lane c: strain Ip90. Lane S: both on left and right in the Figure shows the sizes of the molecular weight standards (x10³).

**Figure 2a and b** Western blot analysis using different monoclonal or polyclonal sera reacting with different epitopes of B. burgdorferi. The immunoblots were made form the same type of SDS-PAGE as in Figure 1, with B31 whole cell preparation in lane A, ACA1 in lane B, and Ip90 in lane C. The different sera used are as follows; In 2A H5332, a monoclonal antibody reacting with an epitope of the OspA protein. 2B) H3TS, an OspA monoclonal antibody reacting with a different epitope than H5332. 2C) H6831, a monoclonal antibody directed against the OspB protein of B. burgdorferi B31. 2D) A polyvalent polyclonal antisera raised against whole cell lysates from the Swedish B. burgdorferi tick strain G152.

**Figure 3a1-3b5** Comparison of the nucleotide sequences of the osp-operons from B. burgdorferi strains B31, ACA1 and Ip90. The OspA sequences form ZS7 and N40 are also included, The OspA and OspB sequences of B. burgdorferi strain B31 are shown in the top row (14). Hyphens indicate homology between the OspA and OspB genes of the different strains, capital letters indicate differences between the Osp genes, and gaps missing (or inserted) bases. The -10 and -35 regions fo the promotor are indicated by bold types and the ribosomal binding sites (Shine Dalgarno sequences) are underlined.

**Figure 4** Determination of the transcriptional start of the B. burgdorferi B31 osp-operon. The figure shows the primer extension of the osp mRNA together with the corresponding sequence ladder of the osp DNA. The -10 region, the transcriptional start base (+1), ribosomal binding site (RBS) and the two first amino acid codons (Met and Lys) are indicated to the right in the figure.

**Figure 5** Comparison of the deduced amino acid sequence of the B. burgdorferi OspA and OspB proteins. al-a2) Comparison of the OspA proteins of strains B31, ZS7, N40, ACA1 and Ip90. All sequences are compared and aligned to the B. burgdorferi B31 sequence (14). Capital letters indicate differences between the strains, hyphens indicate homologies, and gaps indicate missing amino acids. The possible signal peptidase cleavage site are indicated with bold letters in the figure. b1-b2) The deduced amino acid sequence of the OspB proteins of the B. burgdorferi strains ACA1 and Ip90 compared to the OspB protein of B. burgdorferi B31.

**Figure 6 A and B**
A) Analysis by SDS-PAGE of ³H-palmitic acid labelled proteins
B) Analysis by SDS-PAGE and immunoblotting with OspA monoclonal antibody H5332.

Lane 1 E. coli strain DH5α without expression vector, Lane 2 the same strain with the pS151 vector, Lane 3 the same strain with the pS420 vector, Lane 4 molecular weight markers, 18.5, 27.5, 32.5, 49.5, 80 and 106 kD, Lane 5 E. coli strain TG2a without expression vector, Lane 6 the same strain with the sP151 vector, Lane 7 the same strain with the pS420 vector.

### REFERENCES

1. Åsbrink, E. & A. Hovmark. 1985. Successful cultivation of spirochetes from skin lesions of patients with erythema chronicum migrans Afzelius and acrodermatitis chronica atrophicans. Acta. Path. Microbiol. Immunol. Scand. Sect. B, 93: 161-163.
2. Barbour, A.G. 1984. Isolation and cultivation of Lyme disease spirochetes. Yale J. Biol. Med. 57: 71-75.
3. Barbour, A.G. 1984. Immunochemical analysis of Lyme disease spirochetes. Yale J. Biol. Med. 57: 581-586.
4. Barbour, A.G. 1988. Plasmid Analysis of Borrelia burgdorferi, the Lyme Disease Agent. Journal of Clinical Microbiology 26: 475-478.
5. Barbour, A.G. 1989. Antigenic variation in relapsing fever Borrelia species: genetic aspects. In Berg, D.E. & Howe, M.M. (eds): Mobile DNA, Washington, D.C. American Society for Microbiology, pp. 783-789.
6. Barbour, A.G. & C.F. Garon. 1987. Linear plasmids of the bacterium Borrelia burgdorferi have covalently closed ends. Science 237: 409-411.
7. Barbour, A.G., R.A. Heiland & T.R. Howe. 1985. Heterogeneity of major proteins in Lyme disease borreliae: a mMolecular analysis of North American and European isolates. J. Infect. Dis. 152: 478-484.
8. Barbour, A.G. & M.E. Schrumpf. 1986. Polymorphism of major surface proteins of Borrelia burgdorferi. Zentralbl. Bakteriol. Parasitenkd. Infektionskr. Hyg. Abt. 1 Orig. Reihe A. 263: 83-91.
9. Barbour, A.G., S.L. Tessier & S.F. Hayes. 1984. Variation in a major surface protein of Lyme disease spirochetes. Infect. Immun. 45: 94-100.
10. Barbour, A.G., S.L. Tessier & W.J. Todd. 1983. Lyme disease spirochetes and Ixodes ticks share a common surface antigenic determinant defined by a monoclonal antibody. Infect. Immun. 41: 795-804.
11. Barbour, A.G., N. Burman, C.J. Carter, T. Kitten & S. Bergström. 1991. Variable antigen genes of the relapsing fever agent Borrelia hermsii are activated by promoter addition. Mol. Microbiol. 5: 489-493.
12. Barbour, A.G., C.J. Carter, N. Burman, C.S. Freitag, C.F. Garon & S. Bergström. 1991. Tandem insertion sequence-like elements define the expression site for variable antigen genes of Borrelia hermsii. Infect. Immun. 59: 390-397.
13. Bergstrom, S., K. Robbins, M. Koomey & J. Swanson. 1986. Piliation control mechanisms in Neisseria gonorrhoeae. Proc. Natl. Acad. Sci. USA 83: 479-486.
14. Bergström, S., V.G. Bundoc & A.G. Barbour. 1989. Molecular analysis of linear plasmid-encoded major surface proteins, OspA and OspB, of the Lyme disease spirochete Borrelia burgdorferi. Mol. Microbiol. 3: 479-486.
15. Brandt, M.E., B.S. Riley, J.D. Radolf & M.V. Norgard. 1990. Immunogenic integral membrane proteins of Borrelia burgdorferi are lipoproteins. Infect. Immun. 58: 983-991.
16. Bundoc, V.G. & A.G. Barbour. 1989. Clonal polymorphism of outer membrane protein OspB of Borrelia burgdorferi. Infect. Immun. 57: 2733-2741.
17. Burman, N., S. Bergström, B.I. Restrepo & A.G. Barbour. 1990. The variable antigens Vmp7 and Vmp21 of the relapsing fever bacterium Borrelia hermsii are structurally analogous to the VSG proteins of the African trypanosome. Mol. Microbiol. 4: 1715-1726.
18. Craft, J.E., D.K. Fischer, G.T. Shimamoto & A.C. Steere. 1986. Antigens of Borrelia burgdorferi recognized during Lyme disease. Appearance of a new IgM response and expansion of the IgG response late in illness. J. Clin. Invest. 78: 934-939.
19. Fikrig, E., S.W. Barthold, F.S. Kantor & R.A. Flavell. 1990. Protection of mice against the Lyme disease agent by immunizing with recombinant OspA. Science 250: 553-556.
20. Harr, R., P. Fallman, M. Häggström, L. Wahlström & P. Gustafsson. 1986. GENEUS, a computer system for DNA and protein sequence analysis containing an information retrieval system for the EMBL data library. Nucl. Acids Res. 11: 273-284.
21. Hoheisel, J. & F.M. Pohl. 1986. Simplified preparation of unidirectional deletion clones. Nucl. Acids Res. 14: 3605.
22. Howe, T.R., F.W. LaQuier & A.G. Barbour. 1986. Organization of genes encoding two outer membrane proteins of the Lyme disease agent within a single transcriptional unit. Infect. Immun. 54: 207-212.
23. Howe, T.R., L.W. Mayer & A.G. Barbour. 1985. A single recombinant plasmid expressing two major outer surface proteins of the Lyme disease spirochete. Science 227: 645-646.
24. Huynh, T.U., R.A. Young & R.W. Davis. 1985. Construction and screening cDNA libraries in λgt10 and λgt11. In DNA Cloning, Volume 1, ed. Glover, D.M. IRL Press Limited, Oxford, England, pp. 56-110.
25. Inouye, M. & S. Halegoua. 1980. Secretion and membrane localization of proteins in Escherichia coli. Crit. Rev. Biochem. 7: 339-371.
26. Jameson, B.A. & H. Wolf. 1988. The antigenic index: a novel algorithm for predicting antigenic determinants. Comput. Appl. Biosci. 4: 181-186.
27. Kyruchechnikov, V.N., E.I. Korenberg, S.V. Scherbakov, Yu.V. Yovalevsky & M.L. Levin. 1988. Identification of Borrelia isolated in the USSR from Ixodes persulcatus schulze ticks. J. Microbiol. Epidemiol. Immunobiol. 12: 41-44.
28. Loenen, W.A.M. & W.J. Brammer. 1980. A bacteriophage lambda vector for cloning large DNA fragments made with several restriction enzymes. Gene 20: 249-259.
29. Malloy, D.C., R.K. Nauman & H. Paxton. 1990. Detection of Borrelia burgdorferi using the polymerase chain reaction. J. Clin. Microbiol. 28: 1089-1093.
30. Maniatis, T.E., E.F. Fritsch & J. Sambrook. 1982. Molecular cloning: A laboratory manual. Cold Spring Harbor, New York, Cold Spring Harbor, N.Y. USA, Laboratory press.
31. Meier, J.T., M.I. Simon & A.G. Barbour. 1985. Antigenic variation is associated with DNA rearrangements in a relapsing fever borrelia. Cell 41: 403-409.
32. Nielsen, S.L., K.K.Y. Young & A.G. Barbour. 1990. Detection of Borrelia burgdorferi DNA by the polymerase chain reaction. Mol. Cell. Probes. 4: 73-79.
33. Plasterk, R.H.A., M.I. Simon & A.G. Barbour. 1985. Transposition of structural genes to an expression sequence on a linear plasmid causes antigenic variation in the bacterium Borrelia hermsii. Nature 318: 257-263.
34. Postic, D., C. Edlinger, C. Richaud, F. Grimont, Y. Dufresne, P. Perolat, G. Baranton & P.A.D. Grimont. 1990. Two genomic species in Borrelia burgdorferi. Res. Microbiol. 141: 465-475.
35. Rosa, P.A. & T.G. Schwan. 1989. A specific and sensitive assay for the Lyme disease spirochete Borrelia burgdorferi using the polymerase chain reaction. J. Infect. Dis. 160: 1018-1029.
36. Rosa, P.A., D. Hogan & T.G. Schwan. 1991. Polymerase chain reaction analyses identify two distinct classes of Borrelia burgdorferi. J. Clin. Microbiol. 29: 524-532.
37. Rosenberg, M. & O. Court. 1979. Regulatory sequences involved in the promotion and termination of transcription. Ann. Rev. Genet. 13: 256-275.
38. Sanger, F., S. Nicklen & A.R. Coulson. 1977. DNA sequencing with chain-terminating inhibitors. Proc. Natl. Acad. Sci. USA 74: 5463-5467.
39. Saunders, S.E. & J.F. Burke. 1990. Rapid isolation of miniprep. DNA for double strand sequencing. Nucl. Acids Res. 18: 4948.
40. Schaible, U.E., M.D. Kramer, K. Eichman, M. Modolell, C. Museteanu & M.M. Simon. 1990. Monoclonal antibodies specific for the outer surface protein A (OspA) of Borrelia burgdorferi prevent Lyme borreliosis in severe combined immunodeficiency (scid) mice. Proc. Natl. Acad. Sci. USA 87: 3768-3772.
41. Schubach, W.H., S. Mudri, R.J. Dattwyler & B.J. Luft. 1991. Mapping antibody-binding domains of the major outer surface membrane protein (OspA) of Borrelia burgdorferi. Infect. Immun. 59: 1911-1915.
42. Schwan, T.G. & W. Burgdorfer. 1987. Antigenic changes of Borrelia burgdorferi as a result of in vitro cultivation. J. Infect. Dis. 156: 852-853.
43. Schwan, T.G., W. Burgdorfer & C.F. Garon. 1988. Changes in infectivity and plasmid profile of the Lyme disease spirochete, Borrelia burgdorferi, as a result of in vitro cultivation. Infect. Immun. 56: 1831-1836.
44. Stiernstedt, G. 1985. Tick-borne Borrelia infection in Sweden. Scand. J. Infect. Dis. Suppl. 45: 1-70.
45. Wallich. R., U.E. Schaible, M.M. Simon, A. Heiberger & M.D. Kramer. 1989. Cloning and sequencing of the gene encoding the outer surface protein A (OspA) of a European Borrelia burgdorferi isolate. Nucl. Acids Res. 17: 8864.
46. Williams, J.G. & P.J. Mason. 1985. Hybridization in the analysis of RNA. In Nucleic acid hybridization. Hames, B.D. & S.J. Higgins (eds), Oxford: IRL Press, pp. 139-160.
47. Wilske, B., V. Preac-Mursic, G. Schierz, R. Kuhbeck, A.G. Barbour & M. Kramer. 1988. Antigenic variability of Borrelia burgdorferi. Ann. N.Y. Acad. Sci. 539: 126-143.
48. von Gabain, A., J.G. Belasco, J.L. Schottel, A.C.Y. Chang & S.N. Cohen. 1983. Decay of mRNA in Escherichia coli: investigation of the fate of specific segments of transcripts. Proc. Natl. Acad. Sci. USA 80: 653-657.
49. von Heijne, G. 1983. Patterns of amino acids near signal sequence cleavage sites. Eur. J. Biochem. 133: 17-21.
50. Wu, H.C. & M. Tokunaga. 1986. Biogenesis of lipoproteins in bacteria. In Current Topics in Microbiology and Immunology, Vol. 125, pp. 127-157.
51. Arnhem, N. & Levenson, C.H. 36, October 1, 1990 C&EN special report. Polymerase Chain Reaction.
52. Sambrook, J. et al. Molecular Cloning, a laboratory manual.
53. Old, R.W. and Primrose, S.B. Principles of Gene Manipulation, a textbook.
54. Gray, M.R., Colot, H.V., Guarente, L. and Rosbach, M. 1982. Open reading frame cloning: identification, cloning and expression of open reading frame DNA. Proc. Natl. Acad. Sci. USA 79: 6598-6602.
55. Löwenadler, B., Nilsson, B., Abrahmsén, L., Moks, T., Ljungquist, L., Holmgren, E., Paleus, S., Josephson, S., Philipson, L. and Uhlén, M. 1986. Production of specific antibodies against protein A fusions. EMBO J. 5: 2393-2398.
56. Löwenadler, B., Jansson, B., Paleus, S., Holmgren, E., Nilsson, B., Moks, T., Palm, G., Josephson, S., Philipson, L. and Uhlén, M. 1987. A gene fusion system for generating antibodies against short peptides. Gene 58: 87-97.
57. Löwenadler, B., Svennerholm, A.-M., Gidlund, M., Holmgren, E., Krook, K., Svanholm, C., Ulff, S. and Josephson, S. 1990. Enhanced immunogenicity of recombinant peptide fusions containing multiple copies of a heterologous T helper epitope. Eur. J. Immunol. 20: 1541-1545.
58. Löwenadler, B., Lake, M., Elmblad, A., Holmgren, E., Holmgren, J., Karlström, A. and Svennerholm, A.-M. 1991. A recombinant Escherichia coli heat-stable enterotoxin (STa) fusion protein eliciting anti-STa neutralizing antibodies. FEMS Microbiol. Lett. 82: 271-278.
59. Nakamura, K., Masui, Y. and Inouye, M. 1982. Use of a lac promoter-operator fragment as a transcriptional control switch for expression of the constitutive lpp gene in Escherichia coli. J. Mol. Appl. Gen. 1: 289-299.
60. Smith, D.B. and Johnson, K.S. 1988. Single-step purification of polypeptides expressed in Escherichia coli as fusions with glutathione S-transferase. Gene 67: 31-40.
61. Yansura, D.G. 1990. Expression as trpe fusion. In Methods in Enzymology. Ed. Goeddel, D.V. Vol 185: 161-166.
62. Hanahan D. (1983) J. Mol. Biol. 166:557.
63. von Heijne, G. The structure of signal peptides from bacterial lipoproteins. Protein Engineering vol. 2, no. 7 pp. 531-534, 1989.
64. Nakamura K., Inouye M. DNA sequence of the gene for the outer membrane lipoprotein of E. coli: an extremely AT-rich promoter. Cell 1979; 18:1109-17.

## Claims

1. A method for detecting the presence of a *B. burgdorferi* organism in animals, including humans, the method comprising subjecting a specimen from the animal, such as a body fluid, such as blood, serum, cerebrospinal fluid, synovial fluid, pericardial fluid or urine, or a tissue biopsy, to PCR-DNA analysis which
A) comprises using a nucleotide primer sequence which comprises at least 11 nucleotides, the primer sequence being identical to a part of a sequence of OspA and OspB DNA from one of the B. burgdorferi strains B31, lp90 and ACA1, which part of the sequence is different from any part of the sequence of the two other species, the difference being such that the sequence will not anneal to DNA from the two other species, and, upon completion of the PCR DNA-analysis, identifying the detected *B. burgdorferi* organism as belonging to one of the classes I, II and III of *B. burgdorferi*, of which classes B31, Ip90 and ACA1 are reference strains, or
B) comprises using a combination of at least two nucleotide primer sequences, each of which comprises at least 11 nucleotides, one sequence being identical to a part of a sequence from the OspA and OspB DNA from a first *B. burgdorferi* strain B31, another sequence being identical to a part of a sequence from the OspA and OspB DNA from another *B. burgdorferi* strain Ip90, and optionally a third sequence which is identical to a part of a sequence from the third *B. burgdorferi* strain species ACA1 and, upon completion of the PCR DNA-analysis, identifying the detected *B. burgdorferi* organism as belonging to one of the classes I, II and III of *B. burgdorferi* of which classes strains B31, Ip90 and ACA1 are reference strains, or
C) comprises using a nucleotide primer sequence which comprises at least 11 nucleotides, the primer sequence being identical to a part of a sequence of at least two of three OspA and OspB DNA sequences derived from *B. burgdorferi* strains B31, lp90 and ACA1, and, upon completion of the PCR DNA-analysis, identifying the detected *B. burgdorferi* organism as belonging to a group consisting of at least two of the classes I, II and III of *B. burgdorferi*, of which classes strains B31, Ip90 and ACA1 are reference strains.

2. A diagnostic PCR kit for detecting the presence of a *B. burgdorferi* organism in a specimen from an animal, including a human, substantially independently of the individual strain of the *B. burgdorferi* potentially being present in the specimen, and for identifying the detected *B. burgdorferi* organism as belonging to one of the classes I, II and III of *B. burgdorferi*, of which classes B31, Ip90 and ACA1 are reference strains, the kit comprising the following primers or primer combinations
A) a nucleotide primer sequence which comprises at least 11 nucleotides, the primer sequence being identical to a part of a sequence of OspA and OspB DNA from one of the B. burgdorferi strains B31, Ip90 and ACA1, which part of the sequence is different from any part of the sequence of the two other species, the difference being such that the sequence will not anneal to DNA from the two other species, or
B) a combination of at least two nucleotide primer sequences, each of which comprises at least 11 nucleotides, one sequence being identical to a part of a sequence from the OspA and OspB DNA from a first *B. burgdorferi* strain B31, another sequence being identical to a part of a sequence from the OspA and OspB DNA from another *B. burgdorferi* strain Ip90, and optionally a third sequence which is identical to a part of a sequence from the third *B. burgdorferi* strain species ACA1, or
C) a nucleotide primer sequence which comprises at least 11 nucleotides, the primer sequence being identical to a part of a sequence of at least two of three OspA and OspB DNA sequences derived from *B. burgdorferi* strains B31, Ip90 and ACA1.

3. A method according to claim 1C) wherein the primer comprises a sequence which is identical to one of the following sequences (or one of their complementary sequences):

5´-GTATTAAGTTATATTAATAT-3´ | (SEQ ID NO:1, bp 123-142)
5´-AAAAGGAGAATATATTATGA-3´ | (SEQ ID NO:1, bp 584-607)
5´-AAAAATATTTATTGGGAATA-3´ | (SEQ ID NO:1, bp. 776-796)
5´-GGAAAAGCTAAAGAGGTTTTAAAA-3´ | (SEQ ID NO:1, bp. 806-817)
5´-ACTTCAACTTTAACAATTA-3´ | (SEQ ID NO:1, bp. 85-104)
5´-AATAAGGAGAATTTATGA-3´ | (SEQ ID NO:1, bp. 111-130)
5´-AAAAAAACTAAA-3´ | (SEQ ID NO:1, bp. 948-965)

4. A PCR kit according to claim 2C) wherein the primer comprises a sequence which is identical to one of the following sequences (or one of their complementary sequences):

5´-GTATTAAGTTATATTAATAT-3´ | (SEQ ID NO:1, bp 123-142)
5´-AAAAGGAGAATATATTATGA-3´ | (SEQ ID NO:1, bp 584-607)
5´-AAAAATATTTATTGGGAATA-3´ | (SEQ ID NO:1, bp. 776-796)
5´-GGAAAAGCTAAAGAGGTTTTAAAA-3´ | (SEQ ID NO:1, bp. 806-817)
5´-ACTTCAACTTTAACAATTA-3´ | (SEQ ID NO:1, bp. 85-104)
5´-AATAAGGAGAATTTATGA-3´ | (SEQ ID NO:1, bp. 111-130)
5´-AAAAAAACTAAA-3´ | (SEQ ID NO:1, bp. 948-965)

## Patentansprüche

1. Verfahren zum Nachweisen des Vorhandenseins eines *B. burgdorferi*-Organismus in Tieren, einschließlich Menschen, wobei das Verfahren Unterwerfen einer Probe von dem Tier, z.B. eine Körperflüssigkeit wie z.B. Blut, Serum, Liquor cerebrospinalis, Synovia, Herzbeutelflüssigkeit oder Urin, oder eine Gewebebiopsie einer PCR-DNA-Analyse umfasst, welche umfasst:
A) Verwendung einer Nukleotidprimersequenz, die mindestens 11 Nukleotide umfasst, wobei die Primersequenz mit einem Teil einer Sequenz der OspA- und OspB-DNA von einem der B. burgdorferi-Stämme B31, Ip90 und ACA1 identisch ist, dieser Teil der Sequenz zu einem beliebigen Teil der Sequenz der zwei anderen Spezies unterschiedlich ist, der Unterschied so ist, dass die Sequenz sich nicht an DNA aus den zwei anderen Spezies anlagern wird, und bei Abschluss der PCR-DNA-Analyse Identifizierung des nachgewiesenen *B. burgdorferi*-Organismus dahingehend, ob er zu einer der Klassen I, II und III von *B. burgdorferi* gehört, wobei B31, Ip90 und ACA1 Referenzstämme für diese Klassen sind, oder
B) Verwendung einer Kombination aus mindestens zwei Nukleotidprimersequenzen, von denen jede mindestens 11 Nukleotide umfasst, wobei eine Sequenz mit einem Teil einer Sequenz aus der OspA- und OspB-DNA aus einem ersten *B. burgdorferi*-Stamm, B31, identisch ist, eine andere Sequenz mit einem Teil einer Sequenz aus der OspA- und OspB-DNA aus einem anderen *B. burgdorferi*-Stamm, Ip90, und gegebenenfalls einer dritten Sequenz, die mit einem Teil einer Sequenz aus dem dritten *B. burgdorferi*-Stamm Spezies ACA1 identisch ist und bei Abschluss der PCR-DNA-Analyse Identifizierung des nachgewiesenen *B. burgdorferi*-Organismus dahingehend, ob er zu einer der Klassen I, II und III von *B. burgdorferi* gehört, wobei die Stämme B31, Ip90 und ACA1 Referenzstämme für diese Klassen sind, oder
C) Verwendung einer Nukleotidprimersequenz, die mindestens 11 Nukleotide umfasst, wobei die Primersequenz mit einem Teil einer Sequenz von mindestens zwei von drei OspA- und OspB-DNA-Sequenzen, die von den *B. burgdorferi*-Stämmen B31, Ip90 und ACA1 stammen, identisch ist, und bei Abschluss der PCR-DNA-Analyse Identifizierung des nachgewiesenen *B. burgdorferi-*Organismus dahingehend, ob er zu einer Gruppe bestehend aus mindestens zwei der Klassen I, II und III von *B. burgdorferi* gehört, wobei die Stämme B31, Ip90 und ACA1 Referenzstämme für diese Klassen sind.

2. Diagnostischer PCR-Kit zum Nachweisen des Vorhandenseins eines *B. burgdorferi*-Organismus in einer Probe aus einem Tier, einschließlich eines Menschen, im Wesentlichen unabhängig von dem individuellen Stamm *B. burgdorferi*, der möglicherweise in der Probe vorliegt, und zur Identifizierung des nachgewiesenen *B. burgdorferi*-Organismus dahingehend, ob er zu einer der Klassen I, II und III von *B. burgdorferi* gehört, wobei B31, Ip90 und ACA1 Referenzstämme für diese Klassen sind, wobei der Kit die folgenden Primer oder Primerkombinationen umfasst:
A) eine Nukleotidprimersequenz, die mindestens 11 Nukleotide umfasst, wobei die Primersequenz mit einem Teil einer Sequenz von OspA- und OspB-DNA aus einem der *B. burgdorferi*-Stämme B31, Ip90 und ACA1 identisch ist, dieser Teil der Sequenz zu einem beliebigen Teil der Sequenz der zwei anderen Spezies unterschiedlich ist, der Unterschied so ist, dass die Sequenz sich nicht an DNA aus den zwei anderen Spezies anlagern wird, oder
B) eine Kombination aus mindestens zwei Nukleotidprimersequenzen, von denen jede mindestens 11 Nukleotide umfasst, wobei eine Sequenz mit einem Teil einer Sequenz aus der OspA- und OspB-DNA aus einem ersten *B. burgdorferi*-Stamm, B31, identisch ist, eine andere Sequenz mit einem Teil einer Sequenz aus der OspA- und OspB-DNA aus einem anderen *B. burgdorferi*-Stamm, Ip90, identisch ist, und fakultativ einer dritten Sequenz, die mit einem Teil einer Sequenz aus dem dritten *B. burgdorferi*-Stamm Spezies, ACA1, identisch ist, oder
C) eine Nukleotidprimersequenz, die mindestens 11 Nukleotide umfasst, wobei die Primersequenz mit einem Teil einer Sequenz aus mindestens zwei von drei OspA- und OspB-DNA-Sequenzen, die von den *B. burgdorferi*-Stämmen B31, Ip90 und ACA1 stammen, identisch ist.

3. Verfahren nach Anspruch 1C), wobei der Primer eine Sequenz umfasst, die mit einer der folgenden Sequenzen (oder einer ihrer komplementären Sequenzen) identisch ist:

5'-GTATTAAGTTATATTAATAT-3' | (SEQ ID NO:1, bp 123-142)
5'-AAAAGGAGAATATATTATGA-3' | (SEQ ID NO:1, bp 584-607)
5'-AAAAATATTTATTGGGAATA-3' | (SEQ ID NO:1, bp. 776-796)
5'-GGAAAAGCTAAAGAGGTTTTAAAA-3' | (SEQ ID NO:1, bp. 806-817)
5'-ACTTCAACTTTAACAATTA-3' | (SEQ ID NO:1, bp. 85-104)
5'-AATAAGGAGAATTTATGA-3' | (SEQ ID NO:1, bp. 111-130)
5'-AAAAAAACTAAA-3' | (SEQ ID NO:1, bp. 948-965)

4. PCR-Kit nach Anspruch 2C), wobei der Primer eine Sequenz umfasst, die mit einer der folgenden Sequenzen (oder einer ihrer komplementären Sequenzen) identisch ist:

5'-GTATTAAGTTATATTAATAT-3' (SEQ ID NO:1, bp 123-142)
5'-AAAAGGAGAATATATTATGA-3' (SEQ ID NO:1, bp 584-607)
5'-AAAAATATTTATTGGGAATA-3' (SEQ ID NO:1, bp. 776-796)
5'-GGAAAAGCTAAAGAGGTTTTAAAA-3' (SEQ ID NO:1, bp. 806-817)
5'-ACTTCAACTTTAACAATTA-3' (SEQ ID NO:1, bp. 85-104)
5'-AATAAGGAGAATTTATGA-3' (SEQ ID NO:1, bp. 111-130)
5'-AAAAAAACTAAA-3' (SEQ ID NO:1, bp. 948-965)

## Revendications

1. Procédé de détection de la présence d'un organisme du genre *B.burgdorferi* dans des animaux, y compris des êtres humains, le procédé comprenant l'étape consistant à soumettre un échantillon de l'animal, comme un fluide corporel tel que du sang, du sérum, du liquide céphalorachidien, du liquide synovial, du liquide péricardique ou de l'urine, ou une biopsie tissulaire, à une analyse de l'ADN par PCR qui
A) comprend l'utilisation d'une séquence amorce nucléotidique qui compte au moins 11 nucléotides, la séquence amorce étant identique à une partie d'une séquence d'ADN OspA et OspB de l'une des souches de B.burgdorferi B31, Ip90 et ACA1, laquelle partie de la séquence étant différente de toute partie de la séquence des deux autres espèces, la différence étant telle que la séquence ne se liera pas par appariement à l'ADN des deux autres espèces, et une fois l'analyse de l'ADN par PCR terminée, l'identification de l'organisme *B.burgdorferi* détecté comme appartenant à l'une des classes I, II et III de *B.burgdorferi*, dont les souches B31, Ip90 et ACA1 sont les souches de référence, ou
B) comprend l'utilisation d'une association d'au moins deux séquences amorces nucléotidiques, comptant chacune au moins 11 nucléotides, une séquence étant identique à une partie d'une séquence de l'ADN d'OspA et OspB d'une première souche de *B.burgdorferi*, B31, une autre séquence étant identique à une partie d'une séquence de l'ADN d'OspA et OspB d'une autre souche de *B.burgdorferi*, Ip90, et éventuellement d'une troisième séquence identique à une partie d'une séquence de la troisième souche de *B.burgdorferi*, ACA1, et, une fois l'analyse de l'ADN par PCR terminée, l'identification de l'organisme *B.burgdorferi* détecté comme appartenant à l'une des classes I, II et III de *B.burgdorferi*, dont les souches B31, Ip90 et ACA1 sont les souches de référence, ou
C) comprend l'utilisation d'une séquence amorce nucléotidique qui compte au moins 11 nucléotides, la séquence amorce étant identique à une partie d'une séquence d'au moins deux des trois séquences d'ADN OspA et OspB dérivées des souches de B.burgdorferi B31, Ip90 et ACA1, et, une fois l'analyse de l'ADN par PCR terminée, l'identification de l'organisme *B.burgdorferi* détecté comme appartenant à un groupe comprenant au moins deux des classes I, II et III de *B.burgdorferi*, dont les souches B31, Ip90 et ACA1 sont les souches de référence

2. Kit de diagnostic par PCR destiné à détecter la présence d'un organisme du genre *B.burgdorferi* dans un échantillon d'un animal, y compris un être humain, pratiquement indépendamment de la souche individuelle de *B.burgdorferi* potentiellement présente dans l'échantillon, et à identifier l'organisme *B.burgdorferi* détecté comme appartenant à l'une des classes I, II et III de *B.burgdorferi*, dont les souches B31, Ip90 et ACA1 sont les souches de référence, le kit comprenant les amorces ou combinaisons d'amorces suivantes
A) une séquence amorce nucléotidique qui compte au moins 11 nucléotides, la séquence de l'amorce étant identique à une partie d'une séquence d'ADN OspA et OspB de l'une des souches de B.burgdorferi B31, Ip90 et ACA1, ladite partie de la séquence étant différente de toute partie de la séquence des deux autres espèces, la différence étant telle que la séquence ne se liera pas par appariement à l'ADN des deux autres espèces, ou
B) une association d'au moins deux séquences amorces nucléotidiques, comptant chacune au moins 11 nucléotides, une séquence étant identique à une partie d'une séquence de l'ADN d'OspA et OspB d'une première souche de *B.burgdorferi*, B31, une autre séquence étant identique à une partie d'une séquence de l'ADN d'OspA et OspB d'une autre souche de *B.burgdorferi*, Ip90, et d'éventuellement une troisième séquence identique à une partie d'une séquence de la troisième souche de *B.burgdorferi* , ACA1, ou
C) une amorce nucléotidique qui comprend au moins 11 nucléotides, la séquence de l'amorce étant identique à une partie d'une séquence d'au moins deux des trois séquences d'ADN OspA et OspB dérivées des souches de *B.burgdorferi* B31, Ip90 et ACA1.

3. Procédé selon la revendication 1C) dans lequel l'amorce comprend une séquence qui est identique à l'une des séquences suivantes (ou à l'une de leurs séquences complémentaires):

5'-GTATTAAGTTATATTAATAT-3' | (SEQ ID N°1, pb 123-142)
5'-AAAAGGAGAATATATTATGA-3' | (SEQ ID N°1, pb 584-607)
5'-AAAAATATTTATTGGGAATA-3' | (SEQ ID N°1, pb 776-796)
5'-GGAAAAGCTAAAGAGGTTTTAAAA-3' | (SEQ ID N°1, pb 806-817)
5'-ACTTCAACTTTAACAATTA-3' | (SEQ ID N°1, pb 85-104)
5'-AATAAGGAGAATTTATGA-3' | (SEQ ID N°1, pb 110-130)
5'-AAAAAAACTAAA-3' | (SEQ ID N°1, pb 948-965)

4. Kit de PCR selon la revendication 2C) dans lequel l'amorce comprend une séquence qui est identique à l'une des séquences suivantes (ou à l'une de leurs séquences complémentaires):
